Europäisches Patentamt

European Patent Office

Office européen des brevets

(11) Numéro de publication: **0 146 445**
**B1**

(12) **FASCICULE DE BREVET EUROPEEN**

(45) Date de publication du fascicule du brevet: **13.07.88**

(51) Int. Cl.⁴: **A 61 F 2/22**

(21) Numéro de dépôt: **84402316.8**

(22) Date de dépôt: **14.11.84**

(54) Prothèse cardiaque totale comportant deux pompes découplées associées en une unité fonctionnellement indissociable, et valves électrocommandées pour une telle prothèse.

(30) Priorité: **18.11.83 FR 8318368**

(43) Date de publication de la demande:
**26.06.85 Bulletin 85/26**

(45) Mention de la délivrance du brevet:
**13.07.88 Bulletin 88/28**

(84) Etats contractants désignés:
**DE GB IT**

(56) Documents cités:
**EP-A-0 014 130**
**DE-A-2 544 299**
**US-A-4 173 796**

**SCIENTIFIC AMERICAN, vol. 244, no. 1, janvier 1981, pages 66-72, New York, US; R.K. JARVIK: "The total artificial heart"**

**ÖLHYDRAULIK UND PNEUMATIK, vol. 26, no. 2, 1982, pages 114-118, Frankfurt/Main, DE; R. NEUHAUS: "Einsatz eines elektropneumatischen Servoantriebes in der biomedizinischen Technik"**

(73) Titulaire: **AEROSPATIALE Société Nationale Industrielle**
**37, Boulevard de Montmorency**
**F-75781 Paris Cédex 16 (FR)**

(72) Inventeur: **Lapeyre, Didier**
**Chaignes**
**F-27120 Pacy-Sur-Eure (FR)**
Inventeur: **Chareire, Jean-Louis**
**66, rue Aristide Briand**
**F-92300 Levallois (FR)**

(74) Mandataire: **Bonnetat, Christian et al**
**Cabinet PROPI Conseils 23 rue de Léningrad**
**F-75008 Paris (FR)**

Courier Press, Leamington Spa, England.

## Description

La présente invention concerne une prothèse cardiaque totale comprenant deux pompes, respectivement représentatives du coeur droit et du coeur gauche, ainsi qu'un dispositif de commande desdites pompes.

Les maladies cardiaques sont actuellement la première cause de décès dans le monde industrialisé, bien avant le cancer. Un nombre considérable de sujets adultes meurent prématurément d'une défaillance cardiaque irréversible, alors que, par ailleurs, toutes leurs autres fonctions vitales sont parfaitement saines.

Les causes de ce fléau sont multiples et encore obscures pour la plupart. Des mesures préventives ne peuvent donc avoir un effet réellement significatif.

Les techniques chirurgicales de pontages des lésions obstructives des vaisseaux coronariens sont une solution thérapeutique très efficace, puisque plus de 400 000 malades sont opérés dans le monde chaque année. Malheureusement, cette chirurqie reste bien souvent palliative ou insuffisante.

Aux Etats-Unis, seulement, plus de 8000 malades meurent chaque année au cours ou à la suite immédiate d'une intervention chirurgicale sur le coeur; plus de 100 000 malades, de moins de 65 ans d'âqe, meurent dans le même temps des suites immédiates d'un infarctus massif du myocarde; enfin, on peut identifier plus de 50 000 malades de moins de 65 ans qui meurent d'une insuffisance cardiaque terminale invalidante consécutive à un ou plusieurs infarctus itératifs.

La transplantation cardiaque, c'est-à-dire le greffage sur un malade receveur d'un coeur naturel prélevé sur un donneur, connait aujourd'hui un nouveau regain d'intérêt en raison de l'efficacité d'un nouvel agent pharmacologique spécifique réduisant les phénomènes de rejet immunologique. Malheureusement, le manque. de donneurs disponibles pour répondre aux besoins pose un problème insurmontable.

Le nombre de candidats en attente d'une transplantation cardiaque augmente. Nombreux sont ceux qui, parmi eux, décèdent prématurément avant qu'un greffon acceptable puisse leur être proposé.

La greffe d'un coeur artificiel, alternative mécanique de la transplantation cardiaque, semble donc être la seule solution d'avenir.

Pour devenir opérationnelle, cette solution doit cependant se présenter sous une forme cliniquement acceptable et répondre aux normes de performance, de confort, de sécurité et de fiabilité exigées par la communauté médicale internationale.

Plus de 25 années de recherches intensives et plusieurs milliers d'expérimentations sur l'animal en laboratoire permettent de considérer aujourd'hui cette alternative comme réalisable.

L'historique de ces recherches sur le coeur artificiel, l'état de l'art actuel, et les perspectives pour la fin de la décennie de 1980 ont été exposés récemment par John T. WATSON, responsable de ce domaine auprès du Gouvernement Fédéral Américain, dans:

"Past, Present, and Future of Mechanical Circulatory Support"
J. T. WATSON, Chief, Devices and Technology Branch Division of Heart and Vascular Diseases, National Heart, Lung, and Blood Institute NATIONAL INSTITUTES OF HEALTH, Bethesda Maryland, USA.

Third International Symposium on heart substitution. ROMA, ITALY, MAY 17, 1982.

Plusieurs publications récentes font le point de façon plus précise sur la situation actuelle des réalisations et sur les problèmes critiques rencontrés. Il s'agit de:

1. THE ARTIFICIAL HEART, William S. PIERCE Archives of Surgery, Vol. 112, Déc.1977, page 1430-1438

2. CRITERIA FOR HUMAN TOTAL ARTIFICIAL HEART IMPLANTATION BASED ON STEADY STATE ANIMAL DATA. Robert K. JARVIK Trans. American Society For Artificial Internal Organs. Vol. XXIII 1977 page 533-542.

3. APPROACHES TO THE ARTIFICIAL HEART, William S. PIERCE Surgery, 90, 137.1981

4. THE TOTAL ARTIFICAL HEART. Robert K. JARVIK Scientific American, 244. 74. 1981

5. THE ARTIFICIAL HEART William S. PIERCE Thoracic and cardio-vascular Surgery Editor. William W. L. GLENN, M.D. 1983. APPLETON CENTURY CROFTS

6. PRISE EN CHARGE DEFINITIVE DE LA FONCTION CARDIAQUE PAR LE COEUR ARTIFICIEL. Didier LAPEYRE. Compte-rendus des séances de la Société de Biologie du Collège de France. Tome 175. N°5. 1981. p.559

7. JOURNAL OF THE INTERNATIONAL SOCIETY FOR ARTIFICIAL ORGANS. Feb. 1983. CLEVELAND Vol. 7 Number 1, Raven Press.

8. TOTAL ARTIFICIAL HEART IN TWO-STAGED CARDIAC TRANSPLANTATION.
Denton A. COOLEY et Al. Cardio-vascular diseases Bulletin of the Texas Heart Institute Volume 8, Number 3, September 1981

Les deux autres publications suivantes sont d'importance pour la compréhension de la présente invention:

9. SADE, R. M., CASTANEDA A. R. THE DISPENSIBLE RIGHT VENTRICLE Surgery 77: 624-631. 1975

10. TOTAL SUPPORT OF THE CIRCULATION OF A PATIENT WITH POST-CARDIOTOMY STONE-HEART SYNDROME by a PARTIAL ARTIFICIAL HEART (ALVAD) FOR 5 DAYS....
Lancet 1: 1125, 1978. Denton A. Cooley et Al.

Le 2 décembre 1982, la première application humaine de substitution définitive du coeur naturel défaillant par une prothèse cardiaque totale a été faite par l'équipe chirurgicale de SALT-LAKE CITY (De VRIES, JARVIK, OLSEN et Al). Ce premier essai a été largement commenté dans le monde. Le premier homme à coeur artificiel a pu vivre 112 jours avec une prothèse totale à commande pneumatique extra-corporelle, dispositif classique couramment utilisé sur l'animal depuis plus de 15 ans dans tous les laboratoires qui se consacrent à cette recherche.

Malgré quelques incidents, cette première utilisation sur l'homme a démontré de façon définitive qu'il est possible de prendre en charge la fonction circulatoire humaine par des moyens mécaniques et on a établi que la cause du décès du patient n'était pas liée directement à la prothèse cardiaque. Voir à ce sujet:

THE IMPLANTATION OF THE TOTAL ARTIFICIAL HEART FOR THE TREATMENT OF END STAGE CARDIOMYOPATHY. W.C. De Vries and Al. Salt-Lake-City. Utah. 62nd Annual Meeting of the American Association for Thoracic Surgery: ATLANTA, Georgia, April 27, 1983.

L'acquis de l'état de l'art, rappelé dans les publications citées ci-dessus, peut se résumer en deux constatations essentielles:

1 - Depuis 25 ans, tous les expérimentateurs qui ont pu obtenir des survies de longue durée sur l'animal (plusieurs mois) ont utilisé exclusivement une paire de pompes sanguines à membranes remplaçant les deux ventricules du coeur naturel, et un mode d'activation pneumatique, extracorporel.

De très nombreuses solutions ont été proposées pour intégrer le système d'activation dans les deux pompes sanguines. Cependant, tous les dispositifs qui tentent d'intégrer les moyens mécaniques d'activation aux pompes sanguines ont dû être abandonnés car le poids, le volume excessif et le dégagement de chaleur sont incompatibles avec les contraintes anatomiques, physiologiques et biologiques. On a donc été obligé de découpler les pompes sanguines situées dans la cavité péricardique de leur dispositif mécanique d'activation.

Cependant, plusieurs systèmes sont actuellement en développement pour remplacer la console pneumatique externe lourde et encombrante par un convertisseur électro-hydraulique, électro-pneumatique ou électro-mécanique miniaturisé de façon à pouvoir être implanté à proximité des pompes sanguines, soit dans le thorax, soit dans l'abdomen.

Les solutions techniquement les plus réalistes sont celles de type électro-hydraulique (voir les documents US-A-4,173,796 et US-A-4.369.530) ou de type électro-pneumatique.

A ce jour, une seule tentative d'intégration des moyens d'activation a été couronnée de succès. Il s'agit de celle (voir la référence 5 ci-dessus) développée par ROSENBERG et PIERCE mettant en oeuvre un convertisseur électromécanique à plaque de poussée comprimant des poches à sang en élastomères hémocompatibles. Un veau a vécu 225 jours dans de bonnes conditions. Il s'agit d'un record mondial d'une importance historique, car l'animal a vécu normalement avec une prothèse totalement implantée alimentée de l'extérieur par une source d'énergie électrique. La puissance requise n'était que de 10 watts, alors que le poids de l'animal dépassait 180 kgs., soit le double d'un humain adulte standard.

Cependant, le volume de cette prothèse (90O cm3) et son poids (1,1 kg) sont rédhibitoires pour une application humaine et les possibilités de miniaturisation d'un tel appareil paraissent très limitées.

L'installation de pièces mécaniques usinées dans la prothèse cardiaque elle-même, selon une configuration qui respecte la forme, le volume et le poids du coeur naturel parait en effet sur le plan technologique un défi insurmontable.

Certes les volumes sanguins déplacés durant chaque cycle pourraient être réduits à 40 ou 50 cm3, ce qui constituerait un important gain de place. Cependant, cette réduction devrait obligatoirement entrainer une augmentation de la fréquence opérationnelle de base pour maintenir un débit cardiaque acceptable. La compensation obtenue pénaliserait donc gravement la durée de vie des composants.

2 - Compte-tenu des difficultés considérables rencontrées par les équipes de recherches dans le début des années 1970 pour maintenir en vie de façon prolongée des animaux, même dans les cas où l'activation se faisait par l'intermédiaire d'une console pneumatique extra-corporelle, le Ministère de la Santé Américain (NATIONAL INSTITUTES OF HEALTH) a décidé en 1975 de privilégier une autre alternative, à savoir l'assistance ventriculaire gauche.

Dans cette alternative, le coeur naturel malade est laissé en place. Un ventricule artificiel accessoire est branché en parallèle sur la circulation systémique et court-circuite le ventricule gauche malade. Le ventricule gauche est en effet le véritable "coeur". Il assure la perfusion de tous les organes sous un régime de pression dix fois plus élevé que la circulation pulmonaire (ventricule droit).

Il est responsable de la quasi totalité de la pathologie cardiaque.

A l'inverse, il faut rappeler que le ventricule droit ne perfuse que le tissu pulmonaire et que dans les conditions normales sa fonction motrice sur le volume sanguin circulant est très faible.

Le concept de mise en parallèle du ventricule gauche malade par un ventricule d'assistance artificiel est donc attractif d'autant qu'il laisse le coeur naturel en place, ce qui est un procédé beaucoup moins radical que le remplacement total. Ne serait-ce que sur le plan psychologique et émotionnel, l'assistance ventriculaire gauche parait donc a priori une solution plus "pratique".

De façon connue, le ventricule gauche prothétique est MM placé soit dans le thorax, soit sous le diaphragme, dans la cavité abdominale.

Il peut comporter un système d'activation mécanique intégré puisque les contraintes d'encombrement sont beaucoup moins sévères que dans la cavité péricardique.

Pour des raisons de rendement, les dispositifs préférés sont le plus souvent du type à plaque de poussée. Cinq appareils de ce type sont ainsi en développement depuis 1977.

Ces appareils sont décrits par FRANK D. ALTIERI dans un article récent (voir la référence 7 ci-dessus, pages 5 à 20):

"Status of Implantable Energy Systems to Actuate and Control Ventricular Assist Devices"

Deux systèmes intégrés pouvant être alimentés par une source d'énergie isotopique implantée ou par une batterie thermique sont également en développement: University of WASHINGTON (Modified Stirling Engine) et système AEROJET (Stirling Cycle Thermo-Compressor).

Dans le cas de l'utilisation d'une source d'énergie de type électrique, l'énergie est transmise à travers la peau soit de façon PER-cutané, utilisant des matériaux spécifiques bien tolérés par la peau (dérivés du carbone, polycarbonate, P.T.F.E. etc...) soit de façon TRANS-cutanée par induction magnétique à haute fréquence sur un primaire implanté en position sous-cutanée.

L'expérience accumulée: tant en laboratoire qu'en clinique humaine, montre que le concept d'assistance ventriculaire gauche présente en réalité deux inconvénients majeurs qui mettent en cause très sérieusement son utilité potentielle en clinique humaine.

Sur le plan technique par ailleurs, certains points critiques n'ont pas encore trouvé de solution acceptable:

— A elle seule, une pompe gauche accessoire branchée en parallèle sur le ventricule gauche naturel défaillant n'est pas capable de prendre en charge la totalité de la fonction cardiaque.

Plus de 150 tentatives ont été faites sur l'homme, sans résultats probants. Dans quelques cas, de courte durée, le court-circuitage du ventricule gauche a permis à quelques malades en insuffisance ventriculaire gauche aigüe de passer un cap difficile à la suite immédiate d'une intervention chirurgicale sur le coeur.

Ces cas restent malheureusement rares. Bien que le Ministère de la Santé aux Etats-Unis ait autorisé l'utilisation sur l'homme dans plusieurs centres cardiologiques depuis 1979, la méthode n'a pas convaincu les utilisateurs.

Certains de ses promoteurs, tels que DENTON A. COOLEY l'ont même abandonné.

— Pour les malades en insuffisance ventriculaire gauche chronique invalidante (théoriquement la meilleure indication de l'assistance ventriculaire), les cardiologues et chirurgiens cardiaques manifestent une grande réticence à utiliser une méthode lourde, qui peut hâter l'issue fatale, et qui dans tous les cas est soumise à l'alea d'une défaillance de la fonction ventriculaire droite.

De plus, on pense que la décharge du ventricule gauche malade par le ventricule artificiel auxiliaire favoriserait la diminution progressive des performances du ventricule droit. (Voir EFFECTS OF LEFT HEART BY-PASS ON RIGHT VENTRICULAR FUNCTIONS A.T. MIYAMOTO et Al. Vol. XXVIII TRANS AM SOC. ARTIF. INTERN ORGANS 1983, page 543).

— Enfin, deux points techniques critiques restent encore non résolus: (1) la mise en place d'une "crépine", soit dans la pointe du ventricule gauche malade, soit en amont, dans l'oreillette gauche, entraine des phénomènes de gêne mécanique à l'alimentation du ventricule accessoire (plicatures, coudures, pertes de charges liées au calibre insuffisant, coagulation...) (2) Le débit du ventricule gauche auxiliaire doit être asservi sur la pression d'alimentation du ventricule gauche naturel, elle-même fonction du débit du ventricule droit. Dans le cas où le débit veineux de retour baisse, au cours du repos par exemple, le court-circuitage sanguin par le ventricule auxiliaire peut être d'un très faible débit, entrainant dans le conduit d'alimentation du ventricule auxiliaire la formation d'une prolifération cellulaire ("pannus") qui tend peu à peu à l'obstruer, car le phénomène une fois amorcé est auto-entretenu pour des raisons hydrauliques évidentes.

Au total, l'expérience de ces vingt cinq dernières années, aussi bien dans le domaine du remplacement cardiaque total que dans celui de l'assistance ventriculaire gauche apporte les informations suivantes bien connues des hommes de l'art:

1 — Il est aujourd'hui techniquement impossible de coupler dans la cavité péricardique de l'homme, en lieu et place du coeur naturel défaillant, une prothèse cardiaque totale et son système mécanique d'activation, compte-tenu du fait que le coeur naturel de l'homme pèse en moyenne de 300 à 350 g. et celui de la femme de 250 à 300 g. et que lorsque le coeur est plein de sang (diastole), son volume ne dépasse pas 550 cm3.

Certes de nombreux malades cardiaques présentent une augmentation importante du volume cardiaque ("cardiomégalie").

Malheureusement la très grande majorité des candidats au remplacement cardiaque total par le coeur artificiel appartiennent à la catégorie des cardiomyopathes ischiémiques liée à des lésions coronariennes. Ces malades ne présentent pas d'augmentation significative du volume cardiaque.

Une prothèse cardiaque totale ne peut avoir une masse et un volume très supérieurs à la masse et au volume du coeur naturel. Les contraintes d'encombrement ne sont pas seulement d'ordre physique (volume disponible); elles ont en effet des incidences critiques sur le fonctionnement, comme on le verra ci-après.

On est donc obligé de découpler anatomiquement les pompes sanguines intra-péricardiques et les dispositifs mécaniques qui actionnent ces pompes.

2 — Plusieurs milliers d'expérimentations sur l'animal, et quelques unes sur l'homme, ont montré qu'il était possible de placer, en dehors de la cavité péricardique, soit dans le thorax, soit dans la cavité abdomidale, soit en position rétropéritonale, une pompe et ses moyens d'activation.

De tels espaces sont dits "physiologiquement neutres" et ils peuvent recevoir facilement des systèmes mécaniques d'un volume supérieur à 900 cm3, pour une masse pouvant atteindre sans dommages de 1000 à 1200 grammes.

3 — Dans les documents américains US—A—4 173 796, US—A—4 222 127 et US—A—4 369 530, on rappelle, d'une façon générale et non exhaustive, les principaux objectifs techniques à atteindre lorsque l'on réalise une prothèse cardiaque totale normalisée pouvant remplacer efficacement un coeur naturel humain défaillant, sous une forme techniquement acceptable.

On remarquera que certains de ces objectifs ne sont pas encore atteints et constituent des points critiques bien connus de l'homme de l'art.

Ces points critiques doivent cependant être classés selon un ordre de priorité fondamentale, puisque de la solution de certains d'entre eux, dépendent les solutions qui peuvent être apportées aux autres. Cette hiérarchie des priorités doit être exprimée de façon très claire.

Tout d'abord, on doit tenir compte des exigences anatomiques, car elles ont une incidence critique sur la fonction de la prothèse. Une prothèse cardiaque doit bien évidemment reproduire les capacités de pompage, sinon du coeur naturel d'un athlète, du moins celles d'un greffon cardiaque prélevé sur un donneur. Les capacités fonctionnelles doivent permettre en effet à un malade transplanté de se déplacer, d'effectuer des exercices courants tels que la marche, et même la natation. Des études sur des malades ayant subi une transplantation cardiaque ont montré que les capacités ("pumping capacities") d'une prothèse devaient se situer entre les valeurs extrêmes de 3 à 10 l. par minute.

Bien que le débit varie en fonction des demandes métaboliques dans les limites indiquées ci-dessus, il faut noter que le débit moyen chez un adulte standard, se situe autour de 4 l. par minute pendant plus de 95 % du temps. Enfin, la fréquence ne varie que très peu, et reste presque toujours autour de 70 cycles par minute, avec une répartition dans le cycle de l'ordre de 35 % pour la systole et 65 % pour le temps de remplissage, les variations de fréquence se faisant essentiellement par une variation du temps de remplissage, alors que le temps de systole reste relativement constant (à plus ou moins 5 %).

Un autre point fondamental à rappeler, étroitement lié au premier, est une notion physiologique élémentaire, universellement admise aujourd'hui, mais encore controversée au cours de la décennie précédente. Le coeur est un organe-réponse, dont les capacités fonctionnelles doivent répondre aux variations de débit d'alimentation, variations qui sont liées elles-mêmes aux variations de l'activité métabolique de l'organisme. En d'autres termes, la pompe cardiaque est l'esclave et l'organisme le maître. Ce principe fondamental impose un asservissement de débit basé sur les variations du débit de retour, le signal pilote étant la pression sanguine de retour, puisqu'une augmentation des demandes métaboliques de l'organisme se traduit par une baisse de résistance du circuit, entrainant une augmentation du débit de retour. La pompe cardiaque naturelle répond, par des variations de fréquence, du volume pulsé et de la force de contraction, avec une très grande sensibilité, aux variations de la pression d'alimentation et aux variations des résistances de sortie. Cette sensibilité a pu être quantifiée. Elle se situe autour d'un litre par minute et par millimètre de mercure. En pratique donc, à une augmentation de la pression d'alimentation du coeur droit de l'ordre de 10 millimètres de mercure, un coeur artificiel doit répondre par une variation de débit de 3 à 10 litres par minute avec un temps de réponse relativement court, n'excédant pas quelques secondes.

Un troisième point fondamental est encore d'ordre physiologique. L'alimentation de la pompe cardiaque se fait par l'intermédiaire de deux veines de gros calibre, à savoir la veine cave supérieure et la veine cave inférieure, débouchant dans un réservoir d'alimentation formé par l'oreillette droite. De même, l'alimentation du ventricule gauche se fait par l'intermédiaire de quatre veines pulmonaires, à savoir les veines pulmonaires supérieure et inférieure droites et les veines pulmonaires supérieure et inférieure gauches, débouchant dans le réservoir d'alimentation formé par l'oreillette gauche. Ces veines et réservoirs d'alimentation sont très souples et peuvent donc être facilement comprimés si l'encombrement de la prothèse cardiaque est excessif. Ces veines sont par ailleurs des conduits sans rigidité radiale, donc radialement affaissables, ce qui interdit toute aspiration ou dépression active pour faciliter le remplissage. Ces structures particulières d'alimentation représentent donc un point critique essentiel, d'autant plus important que le rapport pression/volume (compliance) du réseau veineux situé en amont est très faible. Une augmentation faible des résistances à l'alimentation de la pompe cardiaque entraine donc une augmentation de la pression dans le réseau, qui se traduit par une très importante augmentation du volume du circuit veineux. Une partie importante du volume circulant peut être ainsi mise en réserve ou retenue dans le circuit veineux, ce qui, bien entendu, diminue d'autant le débit d'arrivée à la pompe sanguine.

Le problème de l'adaptation de la forme, du volume et du poids de la prothèse cardiaque à l'espace laissé libre dans la cavité péricardique par l'ablation du coeur naturel malade n'est donc pas simplement un problème physique, géométrique et volumique. Ces contraintes de forme, de volume et de poids ont en plus, pour les raisons expliquées ci-dessus, une incidence directe et déterminante sur le débit de retour, et donc sur les caractéristiques fonctionnelles de la pompe cardiaque.

Par approches successives et multiples retouches, il a été possible, après plusieurs milliers d'expérimentations sur l'animal, de résoudre de façon acceptable, ce problème d'adaptation de forme, volume et poids sur l'animal, ce qui explique les survies prolongées aujourd'hui obtenues de façon courante en laboratoire.

Cependant, les contraintes anatomiques de l'homme sont radicalement différentes de celles de l'animal et les résultats obtenus par expérimentation animale ne peuvent, dans ce domaine, être transposés à l'homme. Il est clair, en pratique pour le chirurgien cardiaque, que la géométrie de la prothèse cardiaque doit avant toute chose coïncider avec la configuration de l'espace laissé disponible par l'ablation du coeur naturel malade. Il n'est pas acceptable que ce soit au contraire la cavité thoracique du malade qui doive obligatoirement se conformer à la géométrie de la prothèse. Or, actuellement, les candidats à la mise en place d'une prothèse cardiaque sont choisis parmi ceux présentant une cavité péricardique la plus grande possible, afin de pouvoir y loger une prothèse volumineuse.

Encore, doit-on remarquer que ces prothèses volumineuses ne peuvent être couplées directement dans le péricarde avec un système d'activation mécanique. En effet, de tels systèmes d'activation mécaniques augmenteraient encore le volume des prothèses. On est donc obligé de prévoir pour les prothèses, mais en dehors du péricarde, des convertisseurs électro-pneumatiques ou électro-hydrauliques. Mais alors, le rendement global de ces prothèses est relativement faible et l'on doit prévoir une source d'énergie relativement grande (de l'ordre de 10 à 15 Watts). Dans l'état actuel de l'art, dans le domaine des générateurs électriques les plus peformants, une puissance de 10 Watts ne peut être fournie que par une batterie d'une masse supérieure à un kg, si l'on veut obtenir une autonomie complète de plus de 3 heures avec une batterie portable. Il en résulte descontraintes qui constituent certainement un élément d'inconfort pour le patient. De plus, le rendement relativement faible des convertisseurs électro-pneumatiques et électro-hydrauliques entraine un dégagement de chaleur risquant de créer des points chauds sur le convertisseur.

En plus des contraintes anatomo-physiologiques mentionnées ci-dessus, prenant en considération la géométrie, le poids et le volume de la pompe sanguine artificielle devant remplacer le coeur naturel défaillant, il existe bien évidemment d'autres normes qui doivent impérativement être satisfaites pour que le coeur artificiel puisse devenir une réalité acceptable en clinique humaine. Ces normes sont beaucoup mieux explicitées dans l'état de l'art que les contraintes anatomo-physiologiques, pourtant prioritaires.

Ces normes sont de trois ordres, à savoir: biologique, technologique et économique (le coût d'une prothèse doit être aussi bas que possible).

Les normes biologiques concernent l'utilisation de matériaux spécifiques pour les parties de la pompe sanguine qui sont en contact avec le courant sanguin. Ces matériaux doivent être neutres vis-à-vis des phénomènes biologiques naturels qui déclenchent la coagulation du sang, lorsque le sang est mis en contact avec une surface étrangère. Ces matériaux doivent de plus être nontoxiques et ne pas créer de réactions inflammatoires locales. On connait actuellement un certain nombre de matières plastiques souples utilisées avec succès pour la réalisation de pompes à membrane.

L'expérience a montré que ce genre de pompe était celui qui donnait les meilleurs résultats. Cependant, après plusieurs mois d'usage sur l'animal, des calcifications peuvent se produire au niveau des membranes, aux endroits où celles-ci subissent les contraintes mécaniques maximales à chaque cycle de pompage. Ainsi, un facteur important de longévité de ces matériaux mis en contact avec le sang réside dans la limitation des contraintes mécaniques et c'est l'un des objets de l'invention de prévoir une pompe à membranes dans laquelle la membrane subit peu de contraintes mécaniques.

Les normes technologiques sont également connues de l'homme de l'art. Elles définissent le seuil de qualité qui doit être franchi par le coeur artificiel pour pouvoir entrer dans l'arsenal thérapeutique quotidien du chirurgien cardiaque. Elles concernent la fiabilité, la sécurité, les performances et le confort. A ce dernier propos, la prothèse doit présenter un niveau acoustique faible, une absence de vibration ou de phénomène gyroscopique. De plus, la maintenance doit être simple et la miniaturisation suffisante.

Parmi les normes technologiques, le rendement du système est actuellement le point critique déterminant. En effet, compte-tenu des pertes de charge au niveau de la pompe sanguine, essentiellement situées dans les dispositifs valvulaires, compte-tenu des pertes thermo-dynamiques au niveau du convertisseur et du rendement de la conversion électrique au niveau de la transmission transcutanée, le rendement global doit être optimisé de façon à réduire au minimum la quantité d'énergie à fournir.

En effet, pour permettre une autonomie et une liberté de mouvements aux malades, cette énergie doit pouvoir être apportée, au moins de façon temporaire, par une batterie portable. Il est évident que, plus le rendement est élevé, plus l'énergie à fournir est petite et plus l'autonomie de fonctionnement du système est longue pour une masse donnée d'accumulateur. La prothèse selon l'invention est prévue pour pouvoir être alimentée par une énergie de l'ordre de 5 à 7 watts, pour un adulte standard au repos.

De façon générale, l'objet de la présente invention est, en tenant compte des enseignements rappelés ci-dessus, apportés par l'expérience de ces vingt dernières années de recherche sur le coeur artificiel, la réalisation d'une prothèse cardiaque totale permettant de respecter les priorités anatomo-physiologiques et les normes biologiques, technologiques et économiques, dont la liste a été dressée ci-dessus.

La particularité la plus importante de la présente invention se trouve dans la solution du problème critique de l'encombrement des pompes sanguines mises en place dans le péricarde à la place du coeur malade, car cette solution présente des avantages déterminants pour le fonctionnement de la prothèse et permet d'optimaliser la reproduction des performances d'un coeur naturel sain.

A ces fins, selon l'invention, la prothèse cardiaque totale comprenant deux pompes, respectivement représentatives du coeur droit et du coeur gauche, ainsi qu'un dispositif de commande desdites pompes, est remarquable en ce que, d'une part, elle comporte l'unité fonctionnelle indissociable constituée:

- d'un module péricardique, destiné à être logé dans la cavité du coeur naturel à remplacer et enfermé dans une enveloppe étanche portant quatre orifices de raccord destinés respectivement à être raccordés à l'oreillette droite, à l'artère pulmonaire, à l'oreillette gauche et à l'aorte, lesdits orifices de raccord à l'oreillette droite et à l'artère pulmonaire étant pourvus de valves pour servir respectivement d'orifice d'entrée et d'orifice de sortie à une première pompe logée dans ledit module péricardique et destinée à accomplir la fonction du coeur droit du coeur naturel à remplacer;

- d'un module extra-péricardique, destiné à être logé dans un espace physiologiquement neutre du malade receveur et à accomplir la fonction du coeur gauche du coeur naturel à remplacer, ce module extra-péricardique comportant une enveloppe étanche dans laquelle est enfermée une seconde pompe pourvue d'un orifice d'entrée et d'un orifice de sortie, pourvus chacun d'une valve;

— d'une liaison fonctionnelle entre lesdits modules péricardique et extra-péicardique comportant:

— un premier conduit traversant l'enveloppe dudit module péricardique et réunissant l'orifice de celui-ci correspondant à l'oreillette gauche et l'orifice d'entrée de ladite seconde pompe incorporée dans ledit module extra-péricardique;

— un second conduit traversant l'enveloppe dudit module péricardique et réunissant l'orifice de celui-ci correspondant à l'aorte et l'orifice de sortie de ladite seconde pompe incorporée dans ledit module extra-péricardique;

— un troisième conduit établissant une communication gazeuse entre les côtés desdites première et seconde pompes opposés au sang traversant celles-ci;

et en ce que, d'autre part, ledit dispositif de commande actionne lesdites pompes en opposition.

Ainsi, grâce à la structure éclatée, quoique monobloc et indissociable, de la prothèse selon l'invention, seul ledit module péricardique, c'est-à-dire le couer droit, se trouve dans la cavité péricardique. On dispose alors de l'emplacement nécessaire pour y loger une enveloppe enfermant une pompe et son système électro-mécanique d'actionnement, faisant office de ventricule droit.

Aussi, selon une caractéristique importante de la présente invention, ledit module péricardique comporte un système électro-mécanique d'actionnement de ladite première pompe.

En effet, comme il est mentionné par ailleurs, le coeur droit ne fournit qu'une faible différence de pression au sang, de sorte qu'il ne nécessite que peu de puissance. Cette constatation, associée au gain de place dans la cavité péricardique due à l'éloignement du coeur gauche, permet de loger dans ledit module péricardique un système électro-mécanique d'actionnement direct de ladite première pompe, c'est-à-dire un système à rendement élevé. Par contrecoup, la puissance totale nécessaire au fonctionnement de la prothèse selon l'invention est donc réduite. De plus, la transformation de l'énergie électrique en énergie mécanique s'effectuant avec un bon rendement dans ledit système électro-mécanique, peu de chaleur est dégagée par cette conversion, de sorte que le bilan thermique du malade n'est pas perturbé, et que le risque d'apparition de points chauds sur l'enveloppe du module péricardique est inexistant.

De préférence, ladite liaison réunit la partie inférieure de l'enveloppe dudit module péricardique à la partie supérieure de l'enveloppe dudit module extra péricardique.

Avantageusement, l'enveloppe dudit module péricardique présente, au moins approximativement, la forme du coeur naturel malade à remplacer, et sur ladite enveloppe, la disposition des orifices de raccord à l'oreillette droite, à l'artère pulmonaire, à l'oreillette gauche et à l'aorte correspond au moins sensiblement à la disposition naturelle de ces oreillettes et artères. En effet, la place dégagée dans la cavité péricardique par l'éloignement de la pompe faisant office de coeur gauche, permet d'agencer ledit module péricardique de façon que la jonction aux veines et artères soit aussi optimale que possible. Dans ces conditions, la prothèse selon l'invention peut ne perturber en rien le retour sanguin veineux par les veines caves et les veines pulmonaires et il est alors possible de régler la fréquence de battement de la prothèse en fonction de la pression de retour du sang dans le coeur droit, ce qui est particulièrement avantageux. En effet, le coeur droit est le plus sensible pour la régulation du débit sanguin, puisqu'il reçoit le sang non oxygéné de retour de l'organisme avant de l'envoyer dans le poumon. Il est donc particulièrement intéressant de laisser subsister le coeur droit dans la cavité péricardique.

Par suite, selon une autre caractéristique de la présente invention, un capteur est disposé dans ledit module péricardique pour détecter la pression du sang veineux entrant dans ladite première pompe et réguler le fonctionnement de la prothèse en fonction de la pression mesurée.

Par ailleurs, l'éloignement du coeur gauche de la cavité péricardique peut permettre de ménager suffisamment d'espace pour loger, dans ledit module péricardique, les mécanismes de commande de valves électrocommandées.

Il est donc avantageux, notamment en ce qui concerne le rendement, que les valves prévues dans les orifices de raccord à l'oreillette droite et à l'artère pulmonaire soient des valves électrocommandées. En effet, toutes choses égales par ailleurs dans les qualités hydrauliques de valves, la possibilité de commander leur mouvement augmente le rendement global de pompage, notamment en éliminant les fuites à la fermeture.

De préférence, ladite première pompe est du type à membrane et ledit système électro-mécanique est alors du type à plateau-poussoir en contact direct avec ladite membrane. On peut donc optimaliser également les qualités biologiques de la prothèse selon l'invention, en conformant ledit plateau pour qu'il entraine le minimum de contraintes mécaniques à l'origine de la formation de microfissures dans la membrane, ces microfissures étant de façon connue, à l'origine de la dégradation de la qualité de la membrane motrice de la prothèse.

Le capteur, mentionné ci-dessus et destiné à réguler le fonctionnement de la prothèse, peut alors être monté sur la membrane de ladite première pompe.

On remarquera de plus que, en utilisant, ce qui ne présente aucun inconvénient comme on l'a mentionné par ailleurs, un espace physiologiquement neutre, par exemple un volume thoracique ou abdominal, pour y loger ledit module extrapéricardique, l'invention apporte un avantage considérable. En effet, cet espace physiologiquement neutre peut être choisi aisément accessible au prix d'une opération chirurgicale mineure, de sorte que ledit module extra-péricardique, qui se trouve être le module de puissance et donc celui qui est le plus sujet à usure, pourra en cas de panne, être aisément remplacé par un nouveau module à fonctionnement irréprochable.

Pour faciliter un tel échange, il est avantageux que l'enveloppe dudit module extra-péricardique porte des orifices de raccord respectivement reliés à l'orifice d'entrée, à l'orifice de sortie et au côté de ladite seconde pompe opposé au sang et pouvant être facilement branchés et débranchés desdits premier, second et troisième conduits de ladite liaison entre les modules.

Puisque le volume de l'espace physiologiquement neutre choisi peut être relativement important (jusqu'à un litre), il ne se pose aucun problème d'encombrement, et ledit module extrapéricardique peut incorporer également un système électro-mécanique à rendement élevé pour l'actionnement direct de ladite seconde pompe. Celle-ci peut également être du type à membrane, de sorte que le système électro-mécanique associé est alors du type à plateau-poussoir en contact direct avec cette membrane.

De préférence, chacune des deux valves incorporées dans ledit module extra-péricardique est du type à ouverture et fermeture commandées, comme celles prévues pour ledit module péricardique. Il est alors avantageux que le dispositif de commande de la prothèse comporte un microprocesseur commandant les systèmes électromécaniques incorporés dans lesdits modules, ainsi que les quatre valves de ceux-ci. Ce microprocesseur reçoit alors les informations du capteur disposé dans ledit module péricardique et permettant de régler le rythme cardiaque aux besoins du malade.

Selon une caractéristique essentielle de la présente invention, ledit troisième conduit de ladite liaison entre lesdits modules péricardique et extra-péricardique est souple longitudinalement, mais rigide radialement et il enferme lesdits premier et second conduits.

Ainsi, ces premier et second conduits sont protégés mécaniquement par le troisième. Ceci est particulièrement avantageux, du fait que, comme on le verra ci-après, ledit premier conduit doit être très souple, donc très mince, et ne subir aucune perte de charge provoquée. Le second conduit pouvant être plus rigide et plus épais, il résulte de la disposition de l'invention que ledit premier conduit, fragile, est protégé par les deux autres conduits. Comme on le verra également par la suite, ledit premier conduit, associé à l'espace intérieur dudit troisième conduit, fait alors fonction d'oreillette gauche complémentaire. On remarquera que, grâce audit troisième conduit, ladite liaison entre les modules sert de chambre de compliance pour l'ensemble de la prothèse.

Dans ce troisième conduit, on peut faire passer un câble électrique reliant lesdits modules péricardique et extrapéricardique. Ainsi, la commande du système électromécanique et des valves dudit module péricardique peut passer par ledit module extra-péricardique et ledit câble électrique contenu dans la liaison.

Chaque système électro-mécanique peut comporter un moteur électrique et un système bielle-manivelle évitant l'inversion du sens de rotation dudit moteur électrique. De plus, la disposition relative des articulations du plateaupoussoir, de la bielle et de la manivelle peut être avantageusement déterminée pour que les vitesses d'avance et de recul dudit plateau-poussoir soient adaptées aux nécessités physiologiques et ne nécessitent qu'une faible adaptation cyclique de la vitesse du moteur.

Lorsque le module péricardique présente une forme allongée, le plateau-poussoir peut être articulé autour d'un axe dont la direction générale est au moins sensiblement parallèle à la longueur dudit module afin que la surface dudit plateau-poussoir soit aussi grande que possible.

Lesdites valves électro-commandées peuvent être du type à volet circulaire tournant autour d'un de ses diamètres ou coulissant dans son plan. Elles peuvent également être chacune formées de deux volets semi-circulaires articulés autour d'un diamètre commun et pouvant prendre toutes positions entre une première position externe pour laquelle ils sont coplanaires et opposés et une seconde position externe pour laquelle ils sont accolés dos à dos.

Les figures du dessin annexé feront bien comprendre comment l'invention peut être réalisée. Sur ces figures des références identiques désignent des éléments semblables.

La figure 1 montre schématiquement un coeur naturel en liaison avec ses veines et artères principales, en vue antérieure.

La figure 2 illustre schématiquement le principe de la présente invention.

La figure 3 montre schématiquement, en vue extérieure, un exemple de réalisation de la prothèse cardiaque totale selon l'invention.

Les figures 4, 5 et 6 montrent un exemple de réalisation d'un module péricardique pour la prothèse cardiaque conforme à la présente invention, respectivement en vue de face, en vue de gauche et en vue de dessus, avec arrachements partiels.

Les figures 7, 8 et 9 illustrent, en vues schématiques respectivement comparables à celles des figures 4, 5 et 6, une variante de disposition du plateau-poussoir de la pompe à membrane, pour le module péricardique.

La figure 10 est une section transversale de la liaison tubulaire entre les deux modules de la prothèse selon l'invention.

Les figures 11, 12 et 13 montrent un exemple de réalisation d'un module extra-péricardique pour la prothèse cardiaque totale conforme à la présente invention, respectivement en vue de face, en vue de gauche et en vue de dessus, avec arrachements.

Les figures 14 et 15 montrent schématiquement, respectivement en vue en plan avec coupe partielle et en vue de côté, un premier exemple de réalisation de valve électrocommandée pour les modules péricardique et extra-péricardique de la prothèse cardiaque totale selon l'invention.

Les figures 16 et 17 montrent schématiquement, respectivement en vue en plan avec coupe partielle et en coupe diamètrale, un second exemple de réalisation de valve électrocommandée pour la prothèse selon l'invention.

Les figures 18 et 19 illustrent schématiquement, respectivement en vue de dessus et en vue de côté, un troisième exemple de réalisation de valve électrocommandée double, plus spécialement adaptée au module de pompage extrapéricardique de la prothèse cardiaque totale conforme à l'invention.

La figure 20 donne le schéma synoptique de la prothèse selon l'invention.

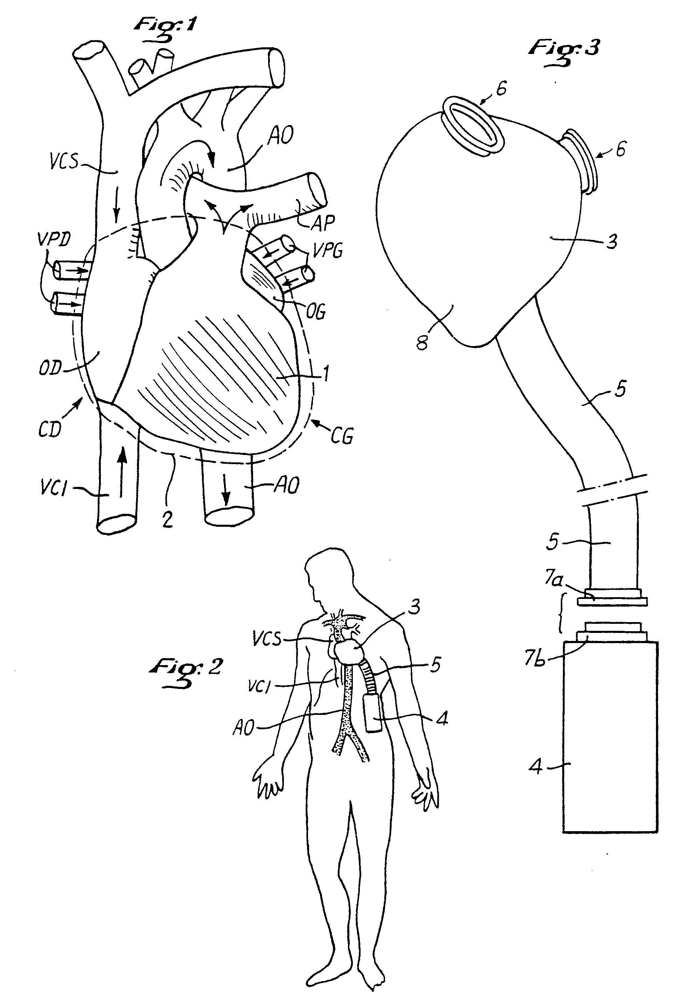

Comme montré sur la figure 1, un coeur humain naturel 1 est logé dans la cavité péricardique 2 (simplement illustrée par un trait tireté 2) et est composé en réalite de deux coeurs distincts, mais solidaires l'un de l'autre, à savoir le coeur droit CD comportant l'oreillette droite OD et le ventricule droit et le coeur gauche CG comportant l'oreillette gauche OG et le ventricule gauche. L'oreillette OD du coeur droit CD reçoit le sang veineux par la veine cave supérieure VCS et par la veine cave inférieure VCI, tandis que le ventricule dudit coeur droit CD fait passer le sang ainsi reçu vers les poumons par l'intermédiaire de l'artère pulmonaire AP.

De même, l'oreillette OG du coeur gauche CG reçoit le sang provenant des poumons par les veines pulmonaires gauches VPG et droites VPD et le ventricule du coeur gauche CG chasse le sang reçu par l'aorte AO.

L'idée de base de la présente invention repose sur la constatation physiologique rappelée ci-dessus, que, quoique constitué de deux pompes CD et CG formant une unité musculaire unique, le coeur 1 est en réalité composé de deux ensembles fonctionnellement indépendants. En effet, sur le plan fonctionnel, le coeur droit CD peut être considéré comme un simple coeur de passage qui pousse une colonne sanguine dont la vitesse d'écoulement est variable, mais jamais nulle, sauf lorsque les fréquences des battements du coeur 1 sont très basses. Lorsque le débit sanguin du système vasculaire augmente par suite d'une augmentation de fréquence de ces battements, la participation du coeur droit CD à la mise en mouvement du sang dans le circuit pulmonaire diminue du fait de l'augmentation de la vitesse, et donc de l'énergie cinétique, du sang parvenant au coeur droit CD. En revanche, le coeur gauche CG, par son puissant ventricule, constitue le coeur proprement dit, c'est-à-dire la pompe propulsive chargée d'assurer la perfusion sanguine de tous les organes et tissus de l'organisme.

De plus, l'idée de base de l'invention s'appuie sur le fait connu de l'homme de l'art que la contraction du ventricule du coeur droit CD et celle du ventricule du coeur gauche CG peuvent être non pas simultanées, mais en opposition de phase.

Comme l'illustre très schématiquement la figure 2, la prothèse cardiaque totale selon l'invention est constituée d'une unité fonctionnellement indissociable constituée de deux modules de pompage 3 et 4 découplés, mais reliés l'un à l'autre par une liaison fonctionnelle tubulaire 5.

Le module de pompage 3, destiné à remplacer le coeur droit CD du coeur naturel 1, est logé dans la cavité péricardique 2. Il comporte quatre embouts de raccord de tout type connu et non représentés, respectivement destinés à le raccorder à l'oreillette droite OD (réservoir des veines caves VCI et VCS), à l'artère pulmonaire AP, à l'oreillette gauche OG (réservoir des veines pulmonaires VPG et VPD) et à l'aorte AO, après coupe de celles-ci et exérèse des ventricules naturels de la cavité péricardique 2.

Le module de pompage 4, destiné à jouer le rôle du coeur gauche CG du coeur naturel 1, est logé à l'extérieur de la cavité péricardique 2, dans un espace physiologiquement neutre, par exemple du thorax ou de l'abdomen.

La liaison 5, qui peut sans inconvénient traverser le diaphragme, comporte trois conduits dont deux relient respectivement le module de pompage 4 aux embouts du module de pompage 3, eux-mêmes respectivement reliés à l'oreillette gauche OG et à l'aorte AO.

Des modes de réalisation avantageux des deux modules de pompage 3 et 4 et de la liaison 5 seront décrits ciaprès plus en détail. Toutefois, dès maintenant, on peut effectuer, au sujet de la prothèse cardiaque totale 3, 4, 5 de l'invention illustrée sur la figure 2, les remarques suivantes:

1) Le gain d'encombrement obtenu par l'élimination du coeur gauche de la cavité péricardique 2 permet:

a) de loger à l'intérieur du module 3 des moyens de pompage, de préférence du type à membrane souple et plaque de poussée, et leurs moyens d'actionnement;

b) éventuellement, d'associer aux embouts de raccord à l'oreillette droite OD et à l'artère pulmonaire AP des électrovannes commandées, plus avantageuses mais plus encombrantes que les simples soupapes usuellement-montées dans les embouts des prothèses cardiaques actuellement connues;

c) d'optimiser le système valvulaire de la prothèse, c'est-à-dire la disposition des embouts de raccord aux oreillettes et artères. Ces embouts peuvent avoir une disposition identique à celle des orifices naturels, de sorte quela prothèse ne perturbe absolument pas le retour sanguin veineux ni à droite, ni à gauche. Par suite, on peut sans difficulté, régler la fréquence des battements de la prothèse d'après la pression de retour du sang au module de pompage 3, simulant le coeur droit CD.

2) Le module de pompage péricardique 3, constituant la prothèse du coeur droit CD, effectue le même travail que celui-ci, c'est-à-dire le plus souvent un simple travail d'accompagnement ou d'appoint pour le passage du sang. Le module de pompage 3 subit donc peu de contraintes et peu d'usure, de sorte que son fonctionnement peut être satisfaisant pendant de longues années. Il n'y a donc aucune contre-indication à loger ce module dans la cavité péricardique 2, dont l'accès, à travers la cage thoracique, nécessite une opération chirurgicale sous circulation extracorporelle, délicate et importante pour le chirurgien, et éprouvante pour le malade. Le module de pompage 3 sera donc, sauf cas exceptionnel, implanté une fois pour toute ou à tout le moins-pour une longue durée dans la cavité péricardique 2.

3) Le module de pompage extra-péricardique 4, simulant le coeur gauche, effectue un travail important et c'est donc lui qui fatigue le plus et est le plus susceptible de nécessiter un remplacement. Il est donc avantageux qu'il puisse être disposé dans un espace physiologiquement neutre, tel que l'abdomen, facilement accessible par une opération chirurgicale bénigne. De plus, ce module 4 se trouvant dans un espace de volume tel que les contraintes d'encombrement sont peu critiques, il peut incorporer ses moyens de pompage, de préférence du type à membrane souple et plaque de poussée et leurs moyens d'actionnement et il peut comporter des valves électro-commandées.

Les remarques 2 et 3 ci-dessus font donc apparaître, d'une part, qu'il n'y a aucun inconvénient à ce que le module de pompage péricardique 3 et la liaison 5 forment un bloc monolithique et, d'autre part, qu'il est avantageux que le module de pompage extra-péricardique 4 et la liaison 5 soit raccordés par un système de raccord rapide. Cette structure est représentée sur la figure 3 sur laquelle on peut voir que le module péricardique 3, pourvu d'embouts de raccord rapide 6 (dont deux seulement sont visibles) pour le relier aux oreillettes OD et OG, à l'artère pulmonaire AP et à l'aorte AO, est solidaire de la liaison 5, alors que celle-ci peut être reliée au module de pompage extra-péricardique 4, par le système de raccord rapide 7a, 7b. Cette liaison 5 relie la partie inférieure du module péricardique 3 à la partie supérieure du module extra-péricardique 4.

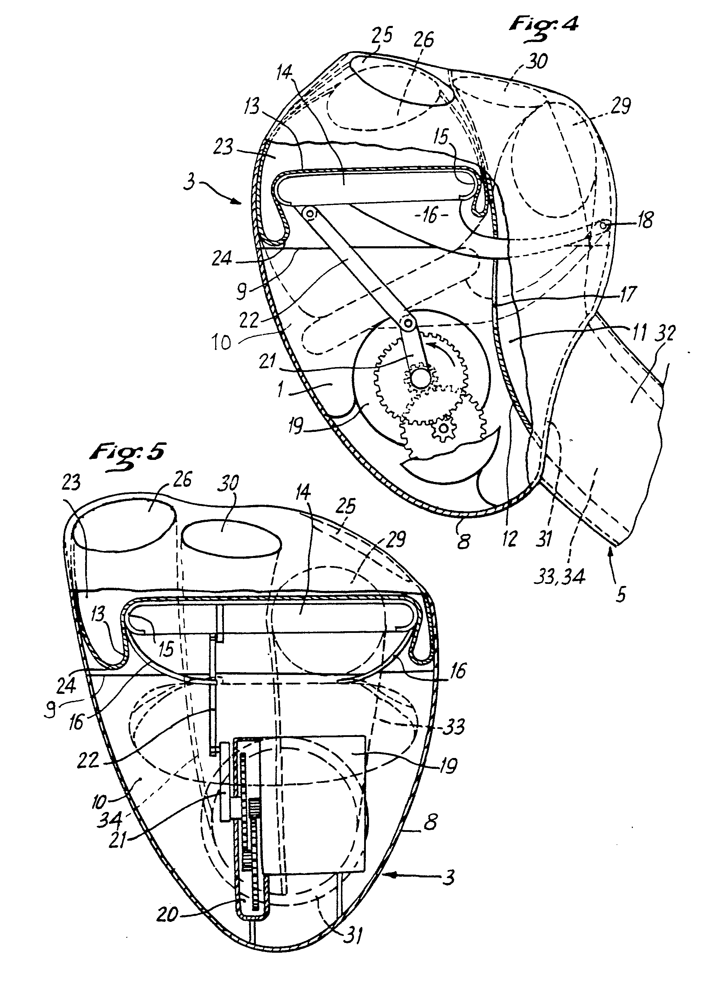

Dans le mode de réalisation du module de pompage péricardique 3, montré par les figures 4, 5 et 6, on a prévu une enveloppe extérieure 8, par exemple en deux parties assemblées le long du plan de joint 9. L'enveloppe 8 présente au moins approximativement la forme d'un coeur naturel et est réalisée en une matière biocompatible, telle que le titane, un composite de carbone, etc...

Le volume intérieur de l'enveloppe 8 est partagé en deux cavités 10 et 11, par une cloison 12 de direction longitudinale. Dans la plus grande des cavités, à savoir la cavité 10, est agencée une pompe, pourvue d'une membrane 13 en une matière hémocompatible, tel qu'un polyuréthane et d'un plateau-poussoir 14 dont le bord périphérique 15 est arrondi et espacé de la paroi interne de la cavité 10. Le plateau-poussoir 14 est muni de bras 16, de direction générale sensiblement parallèle au plan dudit plateau-poussoir. Les bras 16 traversent la paroi 12 par des fentes 17 et sont articulés, à leur extrêmité opposée au plateau-poussoir 14, autour d'un axe 18 parallèle au plan de ce dernier et agencé sur la paroi interne de l'enveloppe 8, dans la cavité 11.

On remarquera que les fentes 17 permettent, en plus du passage des bras 16, la communication gazeuse entre les deux cavités 10 et 11.

Le plateau-poussoir 14 est actionné par un moteur électrique 19, par l'intermédiaire d'un réducteur 20, en boitier étanche lubrifié, ainsi que d'une manivelle 21 et d'une bielle 22.

La membrane 13 est maintenue en place par la paroi interne de l'enveloppe 8 et de la cloison 12 et elle délimite, à l'intérieur de la cavité 10, une poche étanche 23 hémocompatible dans laquelle passe le sang. Afin d'éviter au maximum les contraintes mécaniques imposées à la membrane 13 par le plateau-poussoir 14, celle-ci est simplement posée sur ledit plateau en formant une rigole périphérique 24 autour de celui-ci, cette rigole 24 étant disposée entre le bord arrondi 15 du plateau-poussoir 14 et la paroi interne de la cavité 10. Ainsi, dans le mouvement de la pompe 13, 14, la rigole 24 forme réserve pour la membrane 13, qui peut rouler par rapport audit plateau-poussoir 14, ce qui réduit très sensiblement les contraintes mécaniques appliquées à ladite membrane.

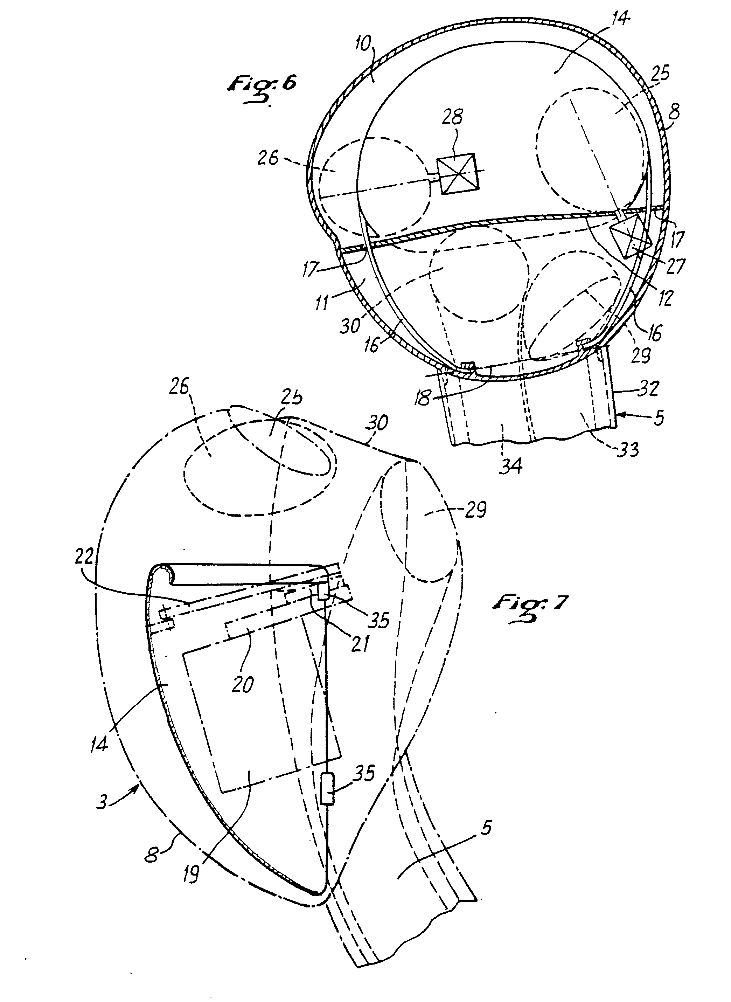

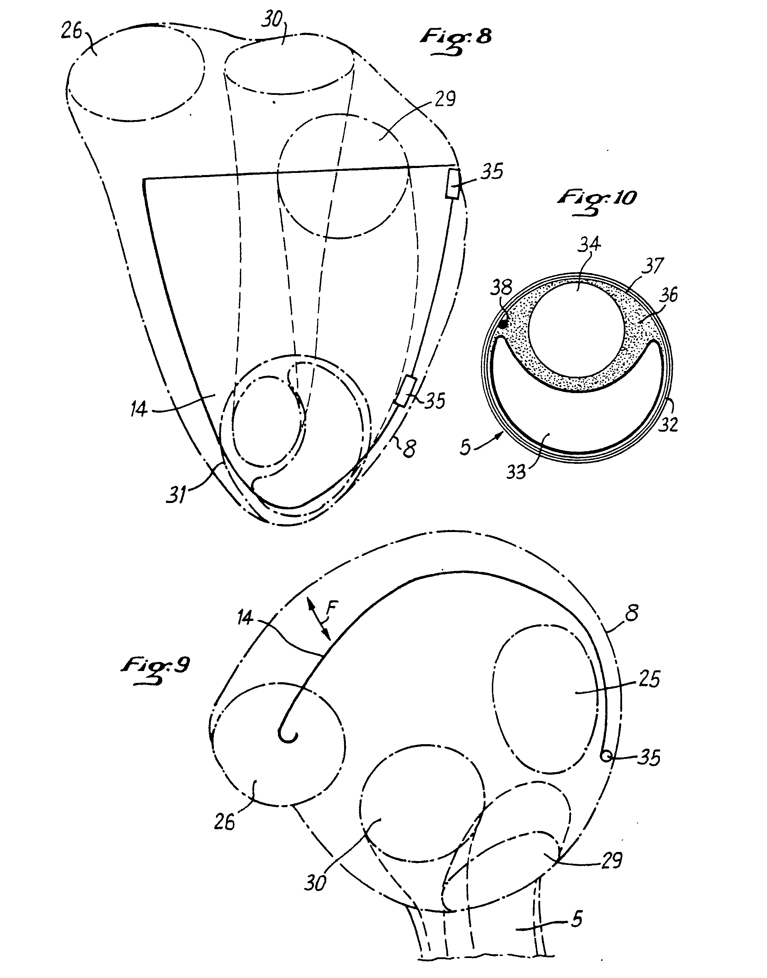

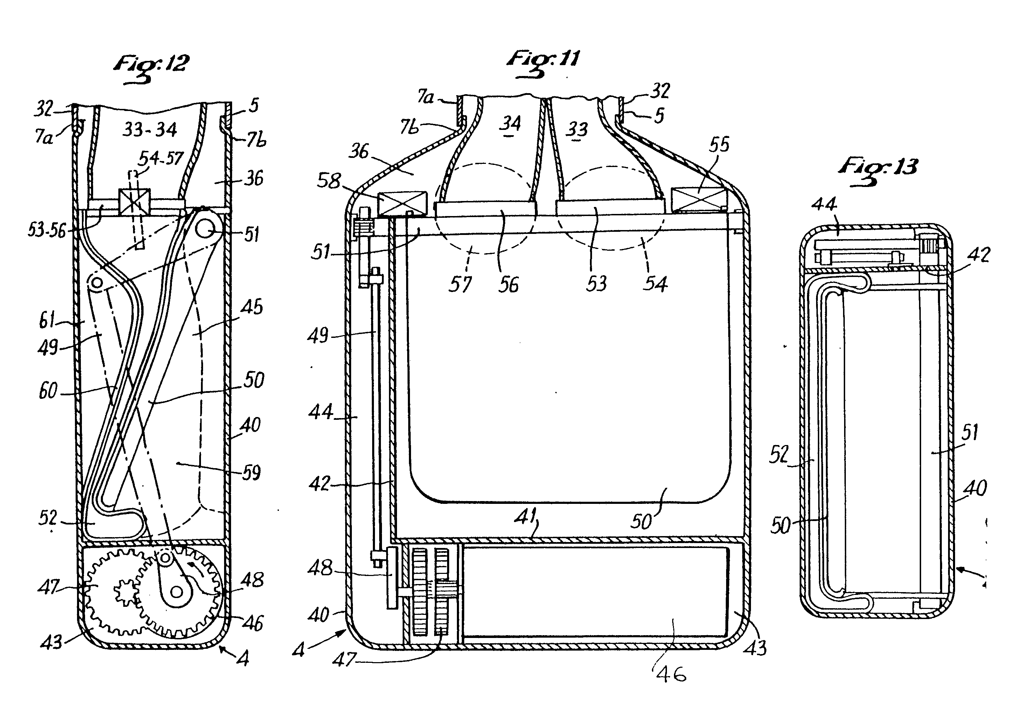

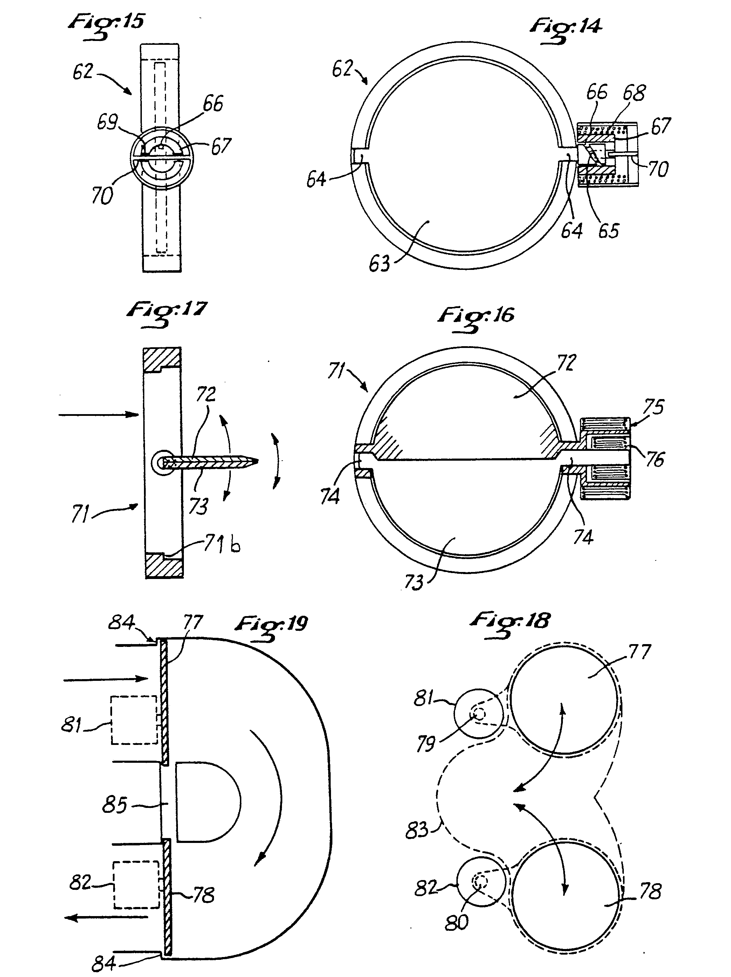

La poche étanche 23 comporte un orifice d'entrée de sang 25 et un orifice de sortie de sang 26, respectivement destinés à être reliés à l'oreillette droite OD et à l'artère pulmonaire AP. Les orifices 25 et 26 sont à cet effet chacun pourvu d'un embout 6 de raccord rapide, qui peut être de tout type connu et qui n'est pas représenté sur les figures 4 à 6 à des fins de clarté. Ces embouts de raccord rapide sont pourvus de valves électrocommandées. Sur la figure 6, on a esquissé schématiquement de telles valves, par leur électro-aimant de commande 27 ou 28. Sur cette figure, on a schématisé des valves du genre à volet pouvant osciller autour d'un diamètre sous l'action desdits électro-aimants.

On décrira par la suite plus en détail des valves électrocommandées pouvant être utilisées dans la prothèse de l'invention; toutefois, on remarquera que ce type de valve n'est pas limitatif, car on pourrait positionner l'axe du volet à un autre endroit ou réaliser des valves en plusieurs parties, par exemple.

Ainsi, sous l'action du moteur 19, le plateau-poussoir 14 et la membrane 13 passent alternativement de la position extrême de sortie de sang (représentée en traits pleins sur les figures 4 et 5) à la position extrême d'entrée de sang (représentée en pointillés sur les figures 4 et 5).

La vitesse du moteur 19 (et donc le rythme de la pompe 13, 14), ainsi que l'ouverture et la fermeture des valves disposées dans les orifices 25 et 26 sont à tout moment imposées par un microprocesseur (non représenté) qui reçoit des informations d'un capteur, également non représenté à des fins de clarté de dessin, et agencé contre la membrane 13.

On remarquera que le système à bielle 22 et manivelle 21, quoique pouvant être remplacé par tout dispositif mécanique approprié, présente les avantages importants suivants:

- il permet d'éviter l'inversion du sens de rotation du moteur 19 à chaque cycle de remplissage et de vidage de la poche 23;

- il permet, grâce aux positions respectives du plateaupoussoir 14, de l'axe 18 et du moteur 19, d'obtenir, pour de faibles variations cycliques de vitesse dudit moteur 19, des différences importantes de vitesse à la montée et à la descente du plateau-poussoir 14. Ainsi, on peut s'approcher des conditions physiologiques idéales de pompage, pour lesquelles le temps de diastole est très différent du temps de systole (généralement $\frac{2}{3}$—$\frac{1}{3}$).

Par ailleurs, la cavité il de l'enveloppe 8 est pourvue d'orifices 29, 30 et 31, traversant la paroi de cette dernière. Les orifices 29 et 30, non équipés de valves, sont chacun pourvus d'un dispositif de raccord rapide 6 (non représentés sur les figures 4 à 6) et sont respectivement destinés à être reliés à l'oreillette gauche OG et à l'aorte AO.

L'orifice 31 est chargé d'établir la communication entre la cavité 11 et l'enveloppe tubulaire 32 de la liaison 5.

De plus, des tuyaux 33 et 34 liés de façon étanche aux embouts 6 des orifices 29 et 30 respectivement, traversent la cavité 11 et passent dans l'enveloppe tubulaire 32 de la liaison 5, à travers l'orifice 31, en direction du module de pompage extra-péricardique 4.

Ainsi, le tuyau 33 prolonge l'oreillette gauche et amène le sang du poumon au module extra-péricardique 4. En revanche, le tuyau 34 refoule le sang, du module extra-péricardique 4 vers l'aorte AO. Le tuyau 33 est réalisé en une matière hémocompatible, tel qu'un polyuréthane, alors que le tuyau 34 est fabriqué par exemple en DACRON ou en TEFLON (marques déposées) comme les prothèses artérielles chirurgicales couramment utilisées en clinique humaine. Toutefois, comme on le verra ci-après, il est avantageux que le tuyau 33 soit très peu rigide afin de pouvoir compléter l'action tampon de l'oreillette gauche OG, alors que le tuyau 34 est inextensible.

La disposition des orifices 25, 26, 29 et 30 est conforme à la disposition naturelle, mais seuls les orifices 25 et 26 sont pourvus de valves. Au total, le volume et la masse du module péricardique 3 sont au plus égaux à ceux du coeur naturel 1 à remplacer.

On remarquera que, grâce aux fentes 17 de la cloison 12, l'espace intérieur 36 de l'enveloppe 32 de la liaison 5 (voir la figure 10) est en liaison avec l'espace mort formé par la partie de la cavité 10, qui est extérieure à la poche 23, et dans laquelle se trouve le mécanisme de pompage 14, 19, 20, 21, 22.

Dans le mode de rélisation des figures 4 à 6, l'axe du moteur 19 est sensiblement transversal à la plus grande dimension de l'enveloppe 8, tandis que le déplacement du plateau-poussoir 14 est sensiblement parallèle à cette plus grande dimension.

Au contraire, dans la variante de réalisation des figures 7, 8 et 9, le déplacement du plateau-poussoir est sensiblement transversal à la plus grande dimension de l'enveloppe 8.

Le plateau-poussoir 14, au lieu d'être plan, est courbe et il est actionné par le moteur 19, dont l'axe est disposé sensiblement parallèle à cette plus grande dimension (le moteur 19, le réducteur 20, la manivelle 21 et la bielle 22 n'ont été indiqués que sur la figue 7). Le plateau-poussoir est articulé au moyen d'articulations 35, lui permettant un débattement figuré par la flèche F sur la figure 9.

Le mode de réalisation des figures 7 à 9 est particulièrement avantageux, car il permet de réaliser un plateau-poussoir 14 de grande surface; il permet donc de limiter l'amplitude de déplacement dudit plateau-poussoir 14 (pour une capacité de pompage identique) et par suite, de réduire les contraintes appliquées à la membrane 13. Celle-ci a donc une durée de vie plus grande.

Sur la figure 10, on a représenté, en coupe transversale, un mode de réalisation avantageux de la liaison tubulaire 5. On peut y voir que le tuyau souple 33 présente une section en forme de croissant pour s'adapter entre l'enveloppe 32 et le tuyau 34 plus rigide.

L'enveloppe 32 présente une souplesse longitudinale suffisante pour lui permettre de s'adapter par flexion, sans coudure ni écrasement au mieux au passage physiologique pouvant exister entre la cavité péricardique 2 dans laquelle est disposé le module de pompage 3 et la cavité extra-péricardique dans laquelle est placé le module de pompage 4. En revanche, l'enveloppe 32 présente une grande rigidité radiale, afin d'éviter toute formation de plis et toute compression extérieure. Cette enveloppe 32 peut par exemple comporter à cet effet, dans sa paroi, un fil spiralé 37 ou analogue. Le diamètre de l'enveloppe tubulaire 32 peut être de l'ordre de 5 cm.

A l'intérieur de ladite enveloppe 32, comme il a été mentionné ci-dessus, passent le tuyau souple 33 prolongeant en une veine pulmonaire commune l'oreillette gauche dans laquelle débouchent les veines pulmonaires gauches et droites, le tuyau plus rigide 34 prolongeant l'aorte et une connexion électrique 38 reliant le module de pompage 3 au module de pompage 4 et à un générateur électrique. La connexion électrique 38 permet l'alimentation et l'asservissement du moteur 19 et des électroaimants 27 et 28 des valves électro-commandées disposées dans les orifices 25 et 26.

Le tuyau souple 33 doit présenter une section supérieure à 8 cm2, pour éviter les pertes de charge, tandis que la section du tyau rigide 34 doit être supériere à 4 cm2.

Les tuyaux 33 et 34 et la connexion électrique 38 laissent subsister, à l'intérieur de l'enveloppe tubulaire 32, l'espace libre 36 mettant en communication gazeuse les espaces morts des deux modules de pompage 3 et 4.

A propos de la structure de la liaison 5, on peut faire les remarques suivantes:

1 - Pour être adaptée à l'oreillette gauche et aux veines pulmonaires, le tuyau 33 doit être très suple et présenter une consistance identique, voisine de la baudruche. Le tuyau 33 est donc très fragile. Cependant, il n'en résulte aucun inconvénient puisque, dans la liaison 5, le tuyau 33 est bien protégé entre les tubes 32 et 34, plus rigides.

2 — L'espace intérieur libre 36, comme il a été dit cidessus et comme il sera monté en regard des figures 11, 12 et 13, met en communication les espaces morts des modules de pompage 3 et 4. Ceux-ci fonctionnant en opposition, ledit espace intérieur libre 36 permet donc au volume gazeux chassé par la pompe d'un desdits modules, de se déplacer vers l'autre de ces derniers.

Il sert donc de chambre de compensation de volume nécessitée par le fonctionnement de pompes à plateau-poussoir. De plus, il permet d'équilibrer les variations de pression atmosphérique puisque le sang contenu dans la veine pulmonaire commune 33 est en équillbre avec la pression atmosphérique. Cet espace intérieur 36 résoud donc de façon élégante le délicat problème de la chambre de "compliance", mentionné par de nombreux auteurs.

3 — La pompe du module de puissance 4 extra-péricardique risque de provoquer des coups de boutoir hydrauliques dans le tuyau 33 prolongeant l'oreillette gauche. Cependant, il n'en résulte aucun inconvénient. En effet, en cas de coups de boutoir hydrauliques, le tuyau 33, dont la consistance est très souple, se dilate radialement, ce qu'il peut faire grâce à l'existence de l'espace libre pneumatique 36. Ainsi, le tuyau 33 en association avec l'espace libre 36 joue le rôle d'un réservoir de sang, à la manière d'une oreillette: il peut donc être considéré comme une oreillette gauche complémentaire.

4 — Eventuellement, pour compléter la fonction tampon de l'espace libre 36, on peut faire en sorte que, à l'intérieur du module péricardique 3, 1 l'enveloppe ou la paroi (ce peut être une partie de l'enveloppe 8) entourant les tuyaux 33 et 34 soit souple, au lieu d'être rigide.

Un mode de réalisation du module de pompage 4 extra-péricardique est représenté sur les figures 11, 12 et 13. Ce mode de réalisation comporte un boitier 40, par exemple de forme parallélépipédique plate, en deux parties.

Dans le boitier 40, des cloisons 41, 42 délimintent des cavités 43, 44 et 45. Dans la cavité inférieure 43 sont disposés un moteur électrique 46 et un réducteur 47, reliés par une manivelle 48 et une bielle 49, logées dans la cavité latérale 44, à un plateau-poussoir 50, agencé dans la cavité supérieure 45. Le plateau-poussoir 50 peut, sous l'action du moteur 46, du réducteur 47, de la manivelle 48 et de la bielle 49, osciller autour d'un axe 51, transversal au boitier 40. Le plateau-poussoir 50 est suspendu en porte-à-faux à son axe 51.

Dans la cavité 45 est disposée une poche étanche 52, reliée de façon étanche à deux orifices, l'un d'entrée, l'autre de sortie, pourvus de systèmes de raccord rapide à valves électro-commandées.

L'orifice d'entrée est pourvu du système de raccord rapide 53 permettant d'y relier aisément le tuyau souple 33. Une valve 54 (représentée en pointillés en position ouverte) est disposée dans ledit système de raccord rapide 53 et est commandée par un électroaimant 55.

De même, l'orifice de sortie est pourvu du système de raccord rapide 56 permettant d'y relier aisément le tuyau plus rigide 34. Une valve 57 (également représentée en pointillés en position ouverte) est agencée dans ledit système de raccord rapide 56 et est commandée par un électro-aimant 58.

Grâce au système de raccord rapide 7a, 7b, l'enveloppe tubulaire 32 de la liaison 5 peut être raccordée au boitier 40, l'espace intérieur libre 36 de la liaison 5 étant alors en communication avec l'espace mort 59 délimité dans la cavité 45, à l'extérieur de la poche étanche 52 et du côté du plateau-poussoir 50 opposé à celle-ci.

On voit ainsi que, grâce aux systèmes de raccord rapide 7a, 7b, 53 et 56, il est aisé de brancher ou de débrancher le module 4 sur la liaison 5 et donc d'établir ou de supprimer la liaison entre les deux modules 3 et 4.

Sur la figure 12, on a représenté en triat plein la position extrême de refoulement (à travers l'embout 56 et le tuyau 34) de la pompe 50-52 et en pointillés la position extrême d'aspiration (à travers le tuyau 33 et l'embout 53) de ladite pompe.

De façon identique à ce qui a été mentionné à propos du module de pompage 3, la membrane de la poche 52 roule sur le plateau-poussoir 50 et le moteur 46 et les électro-aimants 55 et 58 sont commandés par microprocesseur.

Bien entendu, les remarques sur les systèmes d'actionnement par plateau-poussoir et les avantages d'un système bielle-manivelle indiqués ci-dessus, sont les mêmes pour le module 4 extrapéricardique.

A l'intérieur de la cavité 45, du côté opposé au plateaupoussoir 50 par rapport à la poche à sang 52, on peut prévoir une paroi fixe 60 permettant de diminuer le volume mort de ladite poche à sang, afin d'alléger l'ensemble et de remplacer ce volume mort incompressible par un volume mort 61 en communication gazeuse avec les espaces 59 et 36, ce qui diminue d'autant le débit de gaz à travers l'espace 36 de la liaison 5.

La valve électrocommandée 62 montrée par les figures 14 et 15 (susceptible de servir de valve 25, 26, 54 ou 57) comporte un disque circulaire unique 63 pouvant tourner autour d'un axe diamétral 64.

Cet axe 64 porte une rainure hélicoïdale 65 dans laquelle peut coulisser un téton 66 solidaire d'un noyau de fer 67. Le noyau 67 peut coulisser axialement dans un solénoïde 68, si celui-ci est parcouru par un courant électrique. Le noyau 67 ne pouvant tourner autour de son axe, grâce au fait qu'il est pourvu d'une rainure 69 qui coulisse sur la plaquette 70 solidaire du chassis du solénoïde, le déplacement axial de ce noyau 67 provoque donc la rotation du disque 63.

Pour actionner le disque 63, on pourrait également prévoir un aimant permanent (non représenté) fixé sur l'axe 64 et se trouvant placé au centre d'un solénoïde dont l'axe est perpendiculaire à l'axe 64. Ainsi, le passage du courant dans le solénoïde provoque un positionnement de l'aimant permanent de telle sorte que son axe coïncide avec celui du solénoïde, d'où action sur la position de la valve.

En variante, l'axe 64 pourrait simplement être relié au rotor d'un moteur électrique rudimentaire à courant continu.

Le mode de réalisation de valve électrocommandée 71, montré par les figures 16 et 17, comporte deux plaquettes semi-circulaires 72 et 73 pouvant tourner autour du même axe géométrique 74.

Leurs pivots 74 sont donc concentriques.

La plaquette 72 est reliée (pour ses déplacements angulaires) à la cage extérieure 75 d'un moteur électrique.

La plaquette 73 est reliée à la cage intérieure 76 du même moteur.

Par conséquent, le passage du courant électrique provoque un déplacement angulaire de la plaquette 72 par rapport à la plaquette 73, bien que le moteur électrique ne prenne aucun appui angulaire extérieur.

Ainsi, en position ouverte (représentée sur la figure 17), les deux plaquettes semi-circulaires 72 et 73 sont collées l'une contre l'autre, mais peuvent prendre ensemble une position angulaire quelconque par rapport à l'axe de la veine fluide.

De cette façon, c'est le fluide lui-même qui leur donnera la position optimale durant tout l'écoulement, ce qui est un facteur important de réduction des pertes de charges dans la valve. Lors de la fermeture de la valve, il en est de même et le contact des plaquettes 72 et 73 sur la butée 71b n'a généralement Pas lieu au même moment.

Le troisième exemple de réalisation de valves, illustré par les figures 18 et 19 est plus particulièrement destiné au module extra-péricardique 4. Sa structure impose en effet que les deux valves soient dans un même plan. Il présente l'avantage d'éliminer totalement les pertes de charge. Ce mode de réalisation comporte deux volets 77 et 78 articulés sur des axes 79 et 80, qui sont perpendiculaires au plan des valves et parallèles à l'axe des veines fluides.

Des moteurs électriques 81 et 82 ou tout autre système leur permettent de quitter leur position de fermeture pour venir se loger tour à tour dans une cavité 83 dégageant ainsi totalement le passage des veines fluides.

Le contact entre les valves 77 et 78 et les appuis 84 n'est jamais total de manière à ne pas endommager le sang: ce sont les axes 79 et 80 qui subissent en réalité les efforts.

On pourrait éventuellement envisager de n'employer qu'un seul moteur et qu'un seul volet pour obturer tour à tour plus ou moins totalement les deux veines fluides.

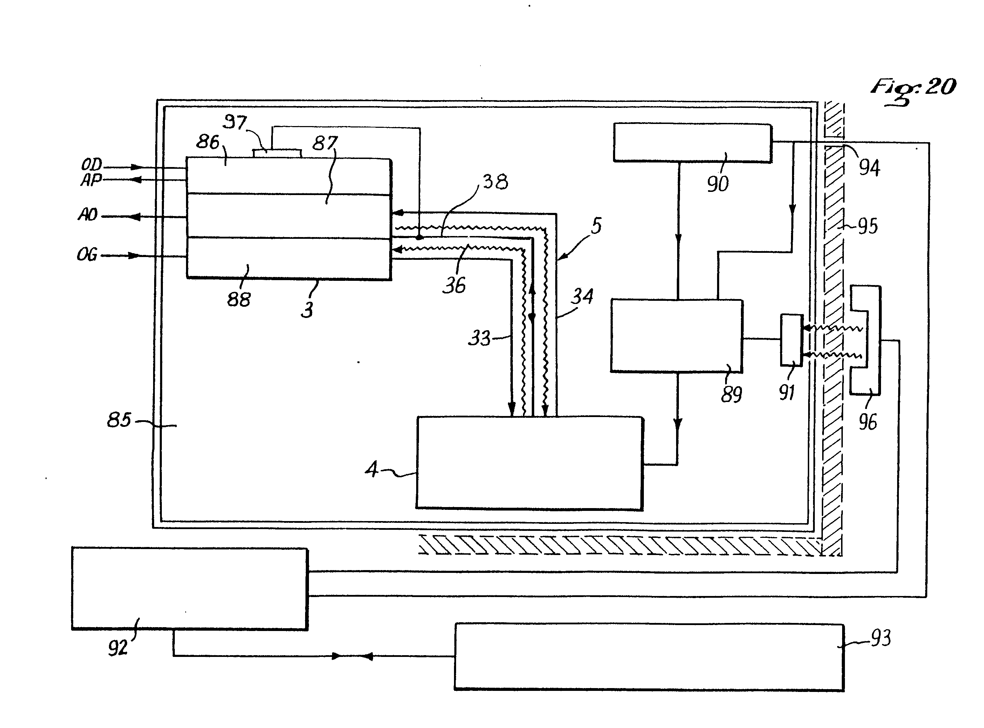

Sur la figure 20, on a représenté le schéma synoptique de la prothèse selon l'invention. L'ensemble 85 des éléments implantés comporte:
— dans la cavité péricardique, le module 3 qui assure la fonction 86 du ventricule de passage entre l'oreillette droite OD et l'artère pulmonaire AP, la fonction 87 de liaison artérielle entre le module 4 et l'aorte AO et la fonction 88 de liaison entre l'oreillette gauche OG et le module 4;
— la liaison 5 comportant le tuyau 33 faisant office de veine pulmonaire commune prolongeant l'oreillette gauche, le tuyau 34 fait office de conduit artériel relié à l'aorte, l'espace 36 faisant office d'oreillette gauche complémentaire et de chambre de compensation de volume commune, et la connexion électrique 38;
— dans une cavité extra-péricardique, le module 4 faisant office du ventricule de puissance gauche;
— une unité électronique 89 servant aux asservissements, au contrôle, etc...
— des batteries secondaires implantables 90 et/ou un récepteur à haute fréquence 91.

Cet ensemble 85 des éléments implantés peut être relié à l'extérieur à un ou plusieurs sous-ensembles portables 92 (électronique, batteries externes rechargeables, etc..) et à une console de diagnostic et de surveillance 93, prenant en compte les paramètres circulatoires et les paramètres de la prothèse.

Entre les éléments implantés 85 et les ensembles 92 et 93 peuvent exister des liaisons percutanées, grâce à des passages 94 à travers la peau 95, et/ou des liaisons transcutanées, grâce à un système d'émission et de réception 91, 96 à haute fréquence.

Sur la figure 20, on a schématisé le capteur 97 disposé dans le module péricardique 3 pour détecter la pression sanguine du retour veineux et piloter le rythme de la prothèse. Pour cela, le capteur 97 est relié à la connexion 38, passant à travers la liaison 5.

On remarquera que, grâce au découplage des pompes obtenu par l'invention, il est possible d'augmenter le volume de sang pompé à chaque cycle, de façon à diminuer la fréquence de pompage et donc à augmenter la durée de vie desdites pompes.

**Revendications**

1. Prothèse cardiaque totale comprenant deux pompes, respectivement représentatives du coeur droit et du coeur gauche, ainsi qu'un dispositif de commande desdites pompes, caractérisée en ce que, d'une part, elle comporte l'unité fonctionnelle indissociable constituée:
- d'un module péricardique (3) destiné à être logé dans la cavité (2) du coeur naturel (1) à remplacer et enfermé dans une enveloppe étanche (8) portant quatre orifices de raccord (25), (26), (29) et (30) destinés respectivement à être raccordés à l'oreillette droite OD, à l'artère pulmonaire AP, à l'oreillette gauche OG et à l'aorte AO, lesdits orifices (25) et (26) de raccord à l'oreillette droite OD et à l'artère pulmonaire AP étant pourvus de valves pour servir respectivement d'orifice d'entrée et d'orifice de sortie à une première pompe (13, 14) logée dans ladite enveloppe (8) et destinée à accomplir la fonction du coeur droit du coeur naturel à remplacer;
- d'un module extra-péricardique (4), destiné à être logé dans un espace physiologiquement neutre du malade receveur et à accomplir la fonction du coeur gauche du coeur naturel (1) à remplacer, ce module extra-péricardique comportant une seconde pompe (50, 52) enfermée dans une enveloppe étanche (40) pourvue d'un orifice d'entrée (53) et d'un orifice de sortie (56), pourvus chacun d'une valve;
— d'une liaison fonctionelle (5) entre lesits modules comportant:
— un premier conduit (33) traversant ladite enveloppe (8) dudit module péricardique (3) et réunissant l'orifice (29) de celui-ci correspondant à l'oreillette gauche OG et l'orifice d'entrée (53) de la seconde pompe (50, 52) incorporée dans ledit module extra-péricardique (4);
— un second conduit (34) traversant ladite enveloppe (8) dudit module péricardique (3) et réunissant l'orifice (30) de celui-ci correspondant à l'aorte AO et l'orifice de sortie (56) de la seconde pompe (50, 52) incorporée dans ledit module extra-péricardique (4);
— un troisième conduit (32) établissant une communication gazeuse entre les côtés desdites première et seconde pompes opposés au sang traversant celles-ci;

et en ce que, d'autre part, ledit dispositif de commande (89, 90, 92, 93) actionne lesdites pompes en opposition.

2. Prothèse cardiaque totale selon la revendication 1, caractérisée en ce que ledit module péricardique (3) comporte un système électromécanique (19, 20, 21, 22) d'actionnement de ladite première pompe (13, 14);

3. Prothèse cardiaque totale selon l'une des revendications 1 ou 2, caractérisée en ce que ladite enveloppe (8) dudit module péricardique (3) présente, au moins approximativement, la forme, le volume et la masse du coeur naturel à remplacer et en ce que, sur ladite enveloppe (8), la disposition des orifices (25, 26, 29, 30) de raccord à l'oreillette droite OD, à l'artère pulmonaire AP, à l'oreillette gauche OG et à l'aorte AO correspond au moins sensiblement à la disposition naturelle de ces oreillettes et artères.

4. Prothèse cardiaque totale selon l'une des revendications 1 à 3, caractérisée en ce qu'un capteur (97) est disposé dans ledit module péricardique (3) pour détecter la pression du sang veineux entrant dans ladite première pompe (13, 14) et réguler le fonctionnement de la prothèse en fonction de la pression sanguine mesurée.

5. Prothèse cardiaque totale selon l'une des revendications 1 à 4, caractérisée en ce que, dans ledit module péricardique (3), les valves prévues dans les orifices (25, 26) de raccord à l'oreillette droite et à l'artère pulmonaire sont du type électro-commandé.

6. Prothèse cardiaque totale selon l'une des revendications 1 à 5, caractérisée en ce que ladite première pompe (13, 14) est du type à membrane et en ce que ledit système électromécanique d'actionnement comporte un plateau-poussoir (14) en contact direct avec ladite membrane.

7. Prothèse cardiaque totale selon les revendications 4 à 6, caractérisée en ce que ledit capteur (97) est monté sur la membrane (13) de ladite première pompe (13, 14).

8. Prothèse cardiaque totale selon l'une quelconque des revendications 1 à 7, caractérisée en ce que ladite liaison (5) réunit la partie inférieure de ladite enveloppe (8) dudit module péricardique (3) à la partie supérieure de ladite enveloppe (40) dudit module extra-péricardique (4).

9. Prothèse cardiaque totale selon l'une quelconque des revendications 1 à 8, caractérisée en ce que ledit module péricardique (3) et ladite liaison (5) sont solidaires l'un de l'autre, tandis que des raccords de jonction amovible (7a, 7b, 53, 56) sont prévus pour raccorder lesdits premier, second et troisième conduits (33, 34 et 32) de ladite liaison et ledit module extra-péricardique (4);

10. Prothèse cardiaque totale selon l'une quelconque des revendications 1 à 5, caractérisée en ce que ledit troisième conduit (32) de ladite liaison (5) entre les modules est souple longitudinalement, mais rigide radialement et enferme lesdits premier et second conduits (33 et 34);

11. Prothése cardiaque totale selon l'une quelconque des revendications 2 ou 5, caractérisée en ce qu'un câble électrique (38), logé dans ledit troisième conduit (32), relie lesdits modules péricardique (3) et extra-péricardique (4);

12. Prothése cardiaque totale selon l'une quelconque des revendications 1 à 11, caractérisée en ce que ladite seconde pompe (50, 52) est du type à membrane et ce que ledit module extra-péricardique (4) incorpore un système électro-mécanique (46, 47, 48, 49) à plateau-poussoir (50) pour l'actionnement de ladite seconde pompe;

13. Prothése cardiaque totale selon l'une quelconque des revendications 1 à 12, caractérisée en ce que chacune des deux valves (54, 57) incorporées audit module extra-péricardique (4) est du type à ouverture et fermeture électrocommandées.

14. Prothèse cardiaque totale selon l'une des revendications 2, 5, 11, 12 et 13, caractérisée en ce que ledit dispositif de commande (89, 90, 92, 93) comporte un microprocesseur commandant les systèmes électromécaniques desdites première et seconde pompes, ainsi que les valves électrocommandées desdits modules péricardique et extra-péricardique.

15. Prothèse cardiaque totale selon la revendication 14, caractérisée en ce que la commande du système électromécanique et des valves dudit module péricardique (3) passe par ledit module extra-péricardique (4) et le câble électrique (38) logé dans ledit troisième conduit (32) de ladite liaison (5).

16. Prothèse cardiaque totale selon l'une des revendications 6 ou 12, caractérisée en ce que ledit système électromécanique comporte un moteur électrique (19 ou 46) et un système bielle-manivelle (21, 22 ou 48, 49) évitant l'inversion de sens de rotation dudit moteur électrique.

17. Prothèse cardiaque totale selon la revendication 16, caractérisée en ce que la disposition relative des articulations du plateau-poussoir, de la bielle et de la manivelle est déterminée pour que les vitesses d'avance et de recul du plateau-poussoir soient adaptées aux nécessités physiologiques et ne nécessitent qu'une faible adaptation cyclique de la vitesse du moteur.

18. Prothèse cardiaque totale selon la revendication 6, dans laquelle le module péricardique (3) présente une forme allongée, caractérisée en ce que ledit plateau-poussoir (14) est animé d'un mouvement de va et vient de direction au moins approximativement parallèle à la longueur dudit module péricardique (3).

19. Prothèse cardiaque totale selon la revendication 6, dans laquelle le module péricardique (3) présente une forme allongée, caractérisée en ce que ledit plateau-poussoir (14) est animé d'un mouvement de va-et-vient de direction au moins approximativement transversale à la longueur dudit module péricardique (3).

20. Prothèse cardiaque totale selon l'une des revendications 18 ou 19, caractérisée en ce que ledit plateau-poussoir (14) est courbe.

21. Prothèse cardiaque selon la revendication 1, caractérisée en ce que au moins une de ses valves est électrocommandée et comporte un volet circulaire (63) mobile autour d'un de ses diamètres.

22. Prothèse cardiaque selon la revendication 1, caractérisée en ce que au moins une de ses valves est électrocommandée et comporte deux volets semi-circulaires (72, 73) articulés autour d'un axe diamètral commun (74) pour pouvoir prendre une position extrême pour laquelle lesdits volets sont coplanaires et opposés et une autre position extrême pour laquelle lesdits volets sont accolés dos à dos.

23. Prothèse cardiaque selon la revendication 1, caractérisée en ce que au moins une de ses valves est électrocommandée et comporte un volet se déplaçant dans son plan d'un mouvement de va-et-vient pour obturer et dégager alternativement deux orifices.

24. Prothèse cardiaque selon la revendication 1, caractérisée en ce que au moins une de ses valves est électrocommandée et comporte deux volets (77, 78) se déplaçant dans leur plan d'un mouvement de va et vient pour obturer et dégager alternativement deux orifices, chaque volet étant associé à l'un desdits orifices.

**Patentansprüche**

1. Vollständige Herzprothese, die zwei Pumpen enthält, jeweils eine als Ersatz für das rechte Herz und die andere für das linke Herz, sowie eine Steuervorrichtung für die pumpen, dadurch gekennzeichnet, daß sie einerseits die unzertrennliche funktionelle Einheit enthält, bestehend aus:
- einem Herzbeutelmodul (3), der dazu bestimmt ist, in die Höhle (2) des zu ersetzenden natürlichen Herzens (1) eingesetzt zu werden, und der in eine dichte Hülle (8) eingeschlossen ist, die vier Verbindungsöffnungen (25), (26), (29) und (30) enthält, die dazu bestimmt sind, jeweils an die rechte Herzvorkammer OD, an die Lungenarterie AP, an die linke Herzvorkammer OG und an die Aorta AO anzuschließen, wobei die Öffnungen (25) und (26) zur rechten Herzvorkammer OD und zur Lungenarterie AP mit Ventilen versehen sind, die jeweils als Eingangs und Ausgangsöffnung dienen für eine erste Pumpe (13, 14), die in der erwähnten Hülle (8) sitzt und dazu bestimmt ist, die Funktion des rechten Herzens des zu ersetzenden natürlichen Herzens zu übernehmen:
— einem Extraherzbeutelmodul (4), der dazu bestimmt ist, in einem physiologisch neutralen Raum des empfangenden Kranken eingesetzt zu werden und der die Funktion des linken Herzens des zu ersetzenden natürlichen Herzens (1) übernimmt, wobei dieser Modul 2 eine zweite Pumpe (50, 52) enthält, die in einer dichten Hülle (40) sitzt, welche eine Eingangsöffnung (53) und eine Ausgangsöffnung (56) enthält, wobei jede mit einem Ventil versehen ist;
— einer funktionellen Verbindung (5) zwischen den Modulen, bestehend aus
— einer ersten Röhre (33) welche die Hülle (8) des Herzbeutelmoduls (3) durchdringt und dessen Öffnung (29) die der linken Herzvorkammer OG entspricht und die Eintrittsöffnung (53) der zweiten Pumpe (50, 52), die in dem Extraherzbeutelmodul (4) eingebaut ist, verbindet;

— eine zweite Röhre (34), welche die Hülle (8) des Herzbeutelmoduls (3) durchdringt und dessen Öffnung (30), welche der Aorta AO entspricht, und die Austrittsöffnung (56) der zweiten Pumpe (50, 52), die in dem Extraherzbeutelmodul (4) eingebaut ist, verbindet;

— eine dritte Röhre (32), welche eine gasförmige Verbindung zwischen den Seiten der ersten und zweiten Pumpe, die dem letztere durchfließenden Blut gegenüberliegen, herstellt; und daß andererseits, die Steuervorrichtung (89, 90, 92, 93) die genannten Pumpen gegenläufig betätigt.

2. Vollständige Herzprothese nach Anspruch 1, dadurch gekennzeichnet, daß das Herzbeutelmodul (3) ein elektro-mechanisches System (19, 20, 21, 22) zur Betätigung der ersten Pumpe (13, 14) enthält.

3. Vollständige Herzprothese nach einem der Ansprüche 1 oder 2, dadurch gekennzeichnet, daß die Hülle (8) des Herzbeutelmoduls (3) mindestens annähernd die Form, das Volumen und das Gewicht des zu ersetzenden Herzens darstellt und daß auf der Hülle (8) die Lage der Öffnungen (25, 26, 29, 30) zum Anschluß an die rechte Herzvorkammer OD, an die Lungenschlagader AP, an die linke Herzvorkammer OG und an die Aorta AO im wesentlichen der natürlichen Lage dieser Herzvorkammern und Schlagadern entspricht.

4. Vollständige Herzprothese nach einem der Ansprüche 1 bis 3, dadurch gekennzeichnet, daß ein Fühler (97) in dem Herzbeutelmodul (3) angebracht ist, um den Druck des in die erste Pumpe eintretenden venösen Bluts aufzuspüren und das Arbeiten der Prothese entsprechend des gemessenen Blutdruckes zu regulieren.

5. Vollständige Herzprothese nach einem der Ansprüche 1 bis 4, dadurch gekennzeichnet, daß im Herzbeutelmodul (3) in den Öffnungen (25, 26) zum Anschluß an die rechte Herzvorkammer und an die Lungenschlagader vorgesehene Ventile elektrisch gesteuert werden.

6. Vollständige Herzprothese nach einem der Ansprüche 1 bis 5, dadurch gekennzeichnet, daß die erste Pumpe (13, 14) eine Membranpumpe ist und daß das elektro-mechanische Betriebssystem eine Druckscheibe (14), die in direktem Kontakt mit der Membrane steht, enthält.

7. Vollständige Herzprothese gemäß den Ansprüchen 4 und 6, dadurch gekennzeichnet, daß der Fühler (97) auf die Membrane (13) der ersten Pumpe (13,14) montiert ist.

8. Vollständige Herzprothese gemäß einem der Ansprüche 1 bis 7, dadurch gekennzeichnet, daß die Verbindung (5) den unteren Teil der Hülle (8) des Herzbeutelmoduls (3) mit dem oberen Teil der Hülle (40) des Extraherzbeutelmoduls (4) verbindet.

9. Vollständige Herzprothese gemäß irgend einem der Ansprüche 1 bis 8, dadurch gekennzeichnet, daß der Herzbeutelmodul (3) und die Verbindung (5) fest miteinander verbunden sind, während abnehmbare Verbindungen (7a, 7b, 53, 56) vorgesehen sind, um die sogenannte erste, zweite und dritte Röhre (33, 34 und 32) der Verbindung und das Modul (4) zu vereinigen.

10. Vollständige Herzprothese gemäß irgend einem der Ansprüche 1 bis 5, dadurch gekennzeichnet, daß die dritte Röhre (32) der Verbindung (5) zwischen den Modulen in Längsrichtung biegsam ist, aber in Radialrichtung steif und die erste und zweite Röhre (33 und 34) enthält.

11. Vollständige Herzprothese gemäß einem der Ansprüche 2 oder 5, dadurch gekennzeichnet, daß ein Elektrokabel (38) in der dritten Röhre (32), das Herzbeutelmodul (3) und das Modul (4) verbindet.

12. Vollständige Herzprothese gemäß irgend einem der Ansprüche 1 bis 11, dadurch gekennzeichnet, daß die zweite Pumpe (50, 52) eine Membranpumpe ist und daß das Extraherzbeutelmodul (4) ein elektromechanisches System (46, 47, 48, 49) mit Druckscheibe (50) für die Betätigung der zweiten Pumpe enthält.

13. Vollständige Herzprothese gemäß einem der Ansprüche 1 bis 12, dadurch gekennzeichnet, daß jedes der beiden Ventile (54, 57), die im Modul (4) enthalten sind, mit elektro-gesteuerter Öffnung und Schließung versehen sind.

14. Vollständige Herzprothese gemäß einem der Ansprüche 2, 5, 11, 12 und 13, dadurch gekennzeichnet, daß die Steuervorrichtung (89, 90, 92, 93) einen Mikroprozessor enthält, der die elektro-mechanischen Systeme der ersten und zweiten Pumpe steuert, sowie die elektrisch gesteuerten Ventile des Herzbeutelmoduls und des Extraherzbeutelmoduls.

15. Vollständige Herzprothese gemäß Anspruch 14, dadurch gekennzeichnet, daß die Steuerung des elektro-mechanischen Systems und der Ventile des Herzbeutelmoduls (3) durch das Modul (4) und das elektrische Kabel (38), das sich in der dritten Röhre (32) der erwähnten Verbindung (5) befindet, läuft.

16. Vollständige Herzprothese gemäß einem der Ansprüche 6 oder 12, dadurch gekennzeichnet, daß das elektro-mechanische System einen elektrischen Motor (19 oder 46) und ein Pleuel-Kurbel-System (21, 22 oder 48, 49) enthält, die die Umkehr der Drehrichtung des elektrischen Motors verhindern.

17. Vollständige Herzprothese gemäß Anspruch 16, dadurch gekennzeichnet, daß die relative Lage der Gelenke der Druckscheibe, der Pleuel und der Kurbel so festgelegt sind, daß die Vorwärts- und Rückwärtsgeschwindigkeiten der Druckscheibe den physiologischen Notwendigkeiten angepaßt werden und nur eine schwache zyklische Anpassung der Motorgeschwindigkeit benötigen.

18. Vollständige Herzprothese gemäß Anspruch 6, in welchem das Herzbeutelmodul (3) eine längliche Form darstellt, dadurch gekennzeichnet, daß die Druckscheibe (14) mit einer Hin- und Herbewegung versehen ist, die ungefähr parallel der Länge des Herzbeutelmoduls (3) ist.

19. Vollständige Herzprothese gemäß Anspruch 6, in welchem das Herzbeutelmodul (3) eine längliche Form hat, dadurch gekennzeichnet, daß die Druckscheibe (14) mit einer Hin- und Herbewegung versehen ist, die ungefähr quer zur Länge des Herzbeutelmoduls (3) verläuft.

20. Vollständige Herzprothese gemäß einem der Ansprüche 18 oder 19, dadurch gekennzeichnet, daß die Druckscheibe (14) gebogen ist.

21. Herzprothese gemäß Anspruch 1, dadurch gekennzeichnet, daß mindestens eines der Ventile elektrisch gesteuert wird und eine runde bewegliche Klappe (63) um einen seiner Durchmesser enthält.

22. Herzprothese gemäß Anspruch 1, dadurch gekennzeichnet, daß mindestens eines der Ventile elektrisch gesteuert wird und zwei halbrunde Klappen (72, 73) enthält, die um eine gemeinsame diametrale Achse (74) beweglich sind, um eine extreme Lage einnehmen zu können, bei welcher die Klappen koplanär und gegenüberliegend sind und eine andere extreme Lage, bei welcher die Klappen·Rücken an Rücken stehen.

23. Herzprothese gemäß Anspruch 1, dadurch gekennzeichnet, daß mindestens eines der Ventile elektrisch gesteuert wird und eine Klappe enthält, die sich in ihrer Ebene hin- und herbewegt, um nacheinander zwei Öffnungen zu verschließen und zu öffnen.

24. Herzprothese gemäß Anspruch 1, dadurch gekennzeichnet, daß mindestens eines der Ventile elektrisch gesteuert wird und zwei Klappen (77, 78) enthält, die sich in ihrer Ebene hin- und herbewegen, um nacheinander zwei Öffnungen zu schließen und zu öffnen, wobei jede der Klappen einer der erwähnten Öffnungen zugeordnet ist.

**Claims**

1. Total artificial heart comprising two pumps, respectively representative of the right heart and of the left heart, as well as a device for controlling said pumps, characterized in that, on the one hand, it comprises the indissociable functional unit constituted by:
— a pericardial module (3) adapted to be housed in the cavity (2) of the natural heart (1) to be replaced and enclosed in a tight envelope (8) comprising four orifices (25, 26, 29 and 30) for connection adapted respectively to be connected to the right atrium OD, to the pulmonary artery AP, to the left atrium OG and to the aorta AO, said orifices (25) and (26) for connection to the right atrium OD and to the pulmonary artery AP being provided with valves in order to serve respectively as input orifice and as output orifice for a first pump (13, 14) housed in said enveloppe (8) and adapted to perform the function of the right heart of the natural heart to be replaced;
— an extra-pericardial module (4) adapted to be housed in a physiologically neutral space in the recipient patient and to perform the function of the left heart of the natural heart (1) to be replaced, this extra-pericardial module comprising a second pump (50, 52) enclosed in a tight envelope (40) provided with an input orifice (53) and an output orifice (56), each provided with a valve;

— a functional link (5) between said modules comprising:
- a first duct (33) passing through said envelope (8) of said pericardial module (3) and connecting the orifice (29) thereof corresponding to the left atrium OG and the input orifice (53) of said second pump (50, 52) incorporated in said extra-pericardial module (4);
— a second duct (34) passing through said envelope (8) of said pericardial module (3) and connecting the orifice (30) thereof corresponding to the aorta AO and the output orifice (56) of the second pump (50, 52) incorporated in said extra-pericardial module (4);
— a third duct (32) establishing a gaseous communication between the sides of said first and second pumps opposite the blood passing therethrough;

and in that, on the other hand, said control device (89, 90, 92, 93) actuates said pumps in opposition.

2. Total artificial heart according to claim 1, characterized in that said pericardial module (3) comprises an electro-mechanical system (19, 20, 21, 22) for actuating said first pump (13, 14).

3. Total artificial heart according to one of claims 1 or 2, characterized in that said envelope (8) of said pericardial module (3) presents, at least approximately, the shape, volume and mass of the natural heart to be replaced, and in that, on said envelope (8), the layout of the orifices (25, 26, 29, 30) for connection to the right atrium OD, to the pulmonary artery AP, to the left atrium OG and to the aorta AO corresponds at least substantially to the natural arrangement of these atria and arteries.

4. Total artificial heart according to one of claims 1 to 3, characterized in that a sensor (97) is disposed in said pericardial module (3) to detect the pressure of the venous blood entering in said first pump (13, 14) and to regulate the functioning of the artificial heart as a function of the blood pressure measured.

5. Total artificial heart according to one of claims 1 to 4, characterized in that in said pericardial module (3), the valves provided in the orifices (25, 26) for connection to the right atrium and to the pulmonary artery are of the electro-controlled type.

6. Total artificial heart according to one of claims 1 to 5, characterized in that said first pump (13, 14) is of the diaphragm type, and in that said electro-mechanical actuating system comprises a thrust plate (14) in direct contact with said diaphragm.

7. Total artificial heart according to claims 4 to 6, characterized in that said sensor (97) is mounted on the diaphragm (13) of said first pump (13, 14).

8. Total artificial heart according to any one of claims 1 to 7, characterized in that said link (5) joins the lower part of said envelope (8) of said pericardial module (3) to the upper part of said envelope (40) of said extra-pericardial module (4).

9. Total artificial heart according to any one of claims 1 to 8, characterized in that said pericardial module (3) and said link (5) are fast with each other, whilst removable joining connections (7a, 7b, 53, 56) are provided to connect said first, second and third ducts (33, 34 and 32) of said link and said extra-pericardial module (4).

10. Total artificial heart according to any one of claims 1 to 5, characterized in that said third duct (32) of said link (5) between the modules is longitudinally supple but radially rigid and encloses said first and second ducts (33 and 34).

11. Total artificial heart according to any one of claims 2 or 5, characterized in that an electrical cable (38), housed in said third duct (32), connects said pericardial (3) and extra-pericardial (4) modules.

12. Total artificial heart according to any one of claims 1 to 11, characterized in that said second pump (50, 52) is of the diaphragm type and in that said extra-pericardial module (4) incorporates an electro-mechanical system (46, 47, 48, 49) with thrust plate (50) for actuating said second pump.

13. Total artificial heart according to any one of claims 1 to 12, characterized in that each of the two valves (54, 57) incorporated in said extra-pericardial module (4) is of the type with electro-controlled opening and closure.

14. Total artificial heart according to claims 2, 5, 11, 12 and 13, characterized in that said control device (89, 90, 92, 93) comprises a microprocessor controlling the electro-mechanical systems of said first and second pumps, as well as the electro-controlled valves of said pericardial and extra-pericardial modules.

15. Total artificial heart according to claim 14, characterized in that the control of the electro-mechanical system and of the valves of said pericardial module (3) passes through said extra-pericardial module (4) and the electrical cable (38) housed in said third duct (32) of said link (5).

16. Total artificial heart according to one of claims 6 or 12, characterized in that said electro-mechanical system comprises an electric motor (19 or 46) and a connecting rod/crank (21, 22 or 48, 49) system avoiding the reversal of the direction of rotation of said electric motor.

17. Total artificial heart according to claim 16, characterized in that the relative layout of the articulations of the thrust plate, the connecting rod and the crank is determined for the speeds of advance and return of the thrust plate to be adapted to the physiological needs and necessitate only a slight cyclic adaptation of the speed of the motor.

18. Total artificial heart according to claim 6, in which the pericardial module (3) presents an elongated form, characterized in that said thrust plate (14) is animated by a reciprocating movement in a direction at least approximately parallel to the length of said pericardial module (3).

19. Total artificial heart according to claim 6, in which the pericardial module (3) presents an elongated form, characterized in that said thrust plate (14) is animated by a reciprocating movement in a direction at least approximately transverse to the length of said pericardial module (3).

20. Total artificial heart according to one of claims 18 or 19, characterized in that said thrust plate (14) is curved.

21. Artificial heart according to claim 1, characterized in that at least one of its valves is electro-controlled and comprises a circular flap (63) mobile about one of its diameters.

22. Artificial heart according to claim 1, characterized in that at least one of its valves is electro-controlled and comprises two semi-circular flaps (72, 73) articulated about a common diametrical axis (74) in order to be able to take an end position for which said flaps are coplanar and opposite, and another end position for which said flaps are joined back to back.

23. Artificial heart according to claim 1, characterized in that at least one of its valves is electro-controlled and comprises a flap moving in its plane in a reciprocating movement in order alternately to obturate and to free two orifices.

24. Artificial heart according to claim 1, characterized in that at least one of its valves is electro-controlled and comprises two flaps (77, 78) moving in their plane in a reciprocating movement in order alternately to obturate and free two orifices, each flap being associated with one of said orifices.

0 146 445

Fig. 1

VCS
AO
VPD
AP
VPG
OG
OD
1
CD
CG
VCI
AO
2

Fig. 2

VCS
3
VCI
5
AO
4

Fig. 3

6
6
3
8
5
5
7a
7b
4

1

0 146 445

Fig. 4

Fig. 5

*Fig. 6*

*Fig. 7*

Fig.8

Fig.10

Fig.9

Fig.13

Fig.11

Fig.12

Fig.15

Fig.14

Fig.17

Fig.16

Fig.19

Fig.18

Fig. 20

0 146 445